# EUROPÄISCHE PATENTANMELDUNG

(11) **EP 4 276 193 A2**
(43) Veröffentlichungstag der Anmeldung: **15.11.2023**
(21) Anmeldenummer: 23192306.1
(22) Anmeldetag: 19.08.2016
(51) Int. Cl.: C12Q 1/68

(54) **ZUSAMMENSETZUNG UND VERFAHREN ZUR HYBRIDISIERUNG**

(30) Priorität: 21.08.2015 EP 15002487; 03.09.2015 EP 15183642; 07.09.2015 EP 15184010; 09.09.2015 EP 15184389
(62) Teilanmeldung aus: 16757611.5
(71) Anmelder: 42 life sciences GmbH & Co. KG, 27572 Bremerhaven (DE)
(72) Erfinder: Hauke, Sven, 28203 Bremen (DE); Marggraf-Rogalla, Piere, 28816 Stuhr (DE)
(74) Vertreter: Ellberg, Nils

(57) **Zusammenfassung**

Die vorliegende Erfindung betrifft eine Zusammensetzung zur Verwendung bei der Hybridisierung, wobei die Zusammensetzung enthält: (a) mindestens eine Hybridisierungssonde ("Komponente (a)"); (b) mindestens ein polares protisches oder polares aprotisches Lösemittel ("Komponente (b)") mit cyclischer Molekülstruktur; und (c) mindestens ein Carbonsäureamid und/oder dessen Salze ("Komponente (c)") in einer Menge von mehr als 10 Vol.-% bezogen auf die Zusammensetzung. Darüber hinaus betrifft die Erfindung auch eine Verwendung dieser Zusammensetzung und Verfahren zur Detektion von Nukleinsäuren und/oder von Chromosomenaberrationen in einer biologischen Probe mittels Hybridisierung unter Verwendung der Zusammensetzung.

## Beschreibung

### Hintergrund

Die vorliegende Erfindung betrifft das technische Gebiet der Nachweisverfahren für Nukleinsäuren, insbesondere DNA und/oder RNA.

Insbesondere betrifft die vorliegende Erfindung eine Zusammensetzung zur Verwendung bei der Hybridisierung, insbesondere zum Nachweis und/oder zur Detektion von Nukleinsäuren, sowie deren erfindungsgemäße Verwendung. Weiterhin betrifft die vorliegende Erfindung ein Verfahren zur Detektion von Nukleinsäuren bzw. von Chromosomenaberrationen. Schließlich betrifft die vorliegende Erfindung ein Kit zur Detektion von Nukleinsäuren bzw. Chromosomenaberrationen.

Vielen Tumorerkrankungen liegen strukturelle und numerische Chromosomenmutationen, wie Translokationen, Inversionen, segmentelle Duplikationen, Deletionen, Insertionen, Duplikationen, Aneuplodien und Amplifikationen, zu Grunde. Der Nachweis dieser Veränderungen als prädiktiver, prognostischer oder differentialdiagnostischer Marker erfolgt in der Regel durch In-Situ-Hybridisierungen (ISH).

Die In-Situ-Hybridisierung beruht auf der Hybridisierung bzw. Paarung komplementärer Basen von Nukleinsäure-Einzelsträngen, insbesondere DNA-Einzelsträngen, so dass spezifische Nukleinsäuresequenzen in einer Probe, insbesondere in einem Gewebe oder Zellpräparat, detektiert werden können. Dazu werden direkt oder indirekt markierte, synthetisch hergestellte Sonden mit Nukleinsäure-Einzelsträngen der Probe hybridisiert und anschließend detektiert.

Zu Detektionszwecken können unter anderem fluoreszenzmarkierte Nukleinsäurefragmente bzw. fluoreszenzmarkierte Hybridisierungssonden (Fluoreszente ISH (FISH)) zum Einsatz kommen. Darüber hinaus können antigenmarkierte Sonden, insbesondere Hapten-markierte Sonden, zum Einsatz kommen, welche anschließend mit Hilfe von Antikörpern durch Farbreaktionen sichtbar gemacht werden, so dass eine lichtmikroskopische Analyse möglich ist ((Hellfeld ISH (BrISH), Chromogene ISH (CISH), Silber ISH (SISH)).

Zur Durchführung von In-Situ-Hybridisierungen wird üblicherweise zunächst eine zu untersuchende biologische Probe, insbesondere ein Präparat vorzugsweise auf Basis von Gewebeschnitten oder zytologischen Präparaten, bereitgestellt. Die Proben werden zur Vorbereitung auf die In-Situ-Hybridisierung auf Objektträgern fixiert und entwässert. Damit die markierten Sonden mit den Nukleinsäuren, insbesondere der RNA oder DNA, in den Zellen bzw. Zellkernen, hybridisieren kann, erfolgt zunächst ein Denaturierungsschritt, so dass die zu detektierende Nukleinsäure bzw. der zu detektierende Nukleinsäureabschnitt und darüber hinaus die eingesetzten Hybridisierungssonden in einzelsträngiger Form in der Probe vorliegen. Dabei ist es sowohl möglich, Probe und Hybridisierungssonde getrennt voneinander als auch gemeinsam im Sinne einer Co-Denaturierung zu denaturieren. Anschließend erfolgt die Hybridisierung der eingesetzten Hybridisierungssonden mit den in der Probe enthaltenen Nukleinsäuren. Die Hybridisierungssonden liegen üblicherweise gelöst bzw. stabilisiert in Hybridisierungslösungen vor, welche zur Hybridisierung auf die zu untersuchenden Proben aufgetragen werden. Die hybridisierten bzw. an die Zielnukleinsäuren gebundenen Hybridisierungssonden können schließlich wie zuvor bereits geschildert detektiert werden.

Wie nachfolgend im Detail geschildert, sind die Hybridisierungsbedingen, welche die Bindungsspezifität der Hybridisierungssonden sowie die Stringenz der Hybridisierung beeinflussen, für den Erhalt gut auswertbarer Ergebnisse entscheidend:
Für die Hybridisierung bzw. den Hybridisierungserfolg sind insbesondere die Denaturierung von Probe und Hybridisierungssonden und anschließende Renaturierung der Nukleinsäuren, insbesondere der DNA, d.h. die Hybridisierung von Nukleinsäureeinzelsträngen in der Probe mit Nukleinsäureeinzelsträngen der Hybridisierungssonden, von zentraler Bedeutung.

Die Auftrennung von doppelsträngigen Nukleinsäuremolekülen in einzelsträngige Nukleinsäuren bzw. die Denaturierung kann insbesondere bei hohen Temperaturen von ungefähr 90 bis 100 °C erfolgen. Derart hohe Temperaturen sind jedoch der Morphologie der biologischen Probe, insbesondere den bevorzugt eingesetzten Gewebeschnitten bzw. Zellen, abträglich.

Um die Morphologie der Proben zu erhalten und darüber hinaus die Stringenz der Hybridisierung zu verbessern, wird im Stand der Technik die In-Situ-Hybridisierung üblicherweise unter Einsatz von formamidhaltigen Lösungen zur Denaturierung der doppelsträngigen Nukleinsäuren in den biologischen Proben durchgeführt. Durch den Einsatz von Formamid in den zur Hybridisierung verwendeten Lösungen wird die Schmelztemperatur von doppelsträngigen Nukleinsäuren, insbesondere DNA-DNA und DNA-RNA Duplexen, welche im Allgemeinen im Bereich von 90 bis 100 °C liegt, auf 65 bis 80 °C herabgesetzt.

Die aus dem Stand der Technik bekannten Hybridisierungslösungen sind jedoch teilweise mit gewissen Nachteilen verbunden, wie nachfolgend geschildert:
Durch den Einsatz von formamidhaltigen Hybridisierungslösungen kann zwar aufgrund des herabgesetzten Schmelzpunktes der DNA die Morphologie der eingesetzten Proben und somit auch das Hybridisierungsergebnis verbessert werden, allerdings sind formamidhaltige Hybridisierungslösungen üblicherweise mit sehr langen Reaktionsdauern verbunden. Denn durch Formamid kommt es zu einer signifikanten Verlangsamung der Renaturierung, d.h. der Anlagerung bzw. Bindung der Hybridisierungssonden einerseits und den in den zu untersuchenden Proben enthaltenen und zu detektierenden Nukleinsäuren andererseits. Im Stand der Technik sind üblicherweise Hybridisierungs- bzw. Renaturierungsdauern von 10 bis 24 Stunden vorgesehen. In Einzelfällen kann sogar eine Hybridisierungs- bzw. Renaturierungsdauer von bis zu 72 Stunden erforderlich sein.

Um das Problem der langen Hybridisierungsdauern zu überwinden, gibt es im Stand der Technik Ansätze, welche ein Ersetzen des Formamids in den Hybridisierungslösungen durch andersartige Lösemittel vorsehen: So werden beispielsweise in der WO 91/02088 A1 Lösemittel auf Basis von Lactamen in Hybridisierungslösungen eingesetzt. Die WO 00/69899 A1 beschreibt Zusammensetzungen zur Stabilisierung von Nukleinsäuren bzw. Nukleinsäureanaloga für biotechnologische Anwendungen, welche von Formamid verschiedene Lösemittel zur Lösung der Nukleinsäuren enthalten. Mit diesen formamidfreien Lösungen werden jedoch oftmals keine zufriedenstellenden Signalmuster bzw. gut auswertbaren Signalmuster erzielt.

Darüber hinaus weisen die bislang im Stand der Technik bekannten Hybridisierungslösungen, welche die für In-Situ-Hybridisierungen erforderlichen lokusspezifischen Hybridisierungssonden enthalten, weitere Nachteile, wie nachfolgend geschildert, auf:
So werden mit den bekannten Hybridisierungslösungen im Rahmen der Durchführung von In-Situ-Hybridisierungen als Schnelltests, bei welchen schon mit kurzen Hybridisierungsdauern gut auswertbare Signalmuster erzeugt werden, oftmals keine starken Signale erhalten, obwohl ein diesbezüglicher Bedarf, z.B. zur Durchführung von Schnelltests in der Prä- bzw. Postnataldiagnostik oder für die Tumorzytogenetik, besteht.

Darüber hinaus eignen sich die aus dem Stand der Technik bekannten Hybridisierungslösungen kaum oder nur in sehr eingeschränktem Maße für einen Einsatz in automatisierten In-Situ-Hybridisierungsverfahren. Im Vergleich zu nichtautomatischen bzw. manuell durchgeführten In-Situ-Hybridisierungsverfahren müssen bei automatisierten Verfahren größere Mengen bzw. Volumina an Hybridisierungslösung auf die Proben aufgetragen werden. Mit den im Stand der Technik bekannten Hybridisierungslösungen ist es bislang nicht möglich, die für die In-Situ-Hybridisierung erforderlichen Mengen an Hybridisierungssonden auch in hochvolumigen Lösungen bzw. in starker Verdünnung derart zu lösen bzw. zu stabilisieren, dass gute Hybridisierungsergebnisse erzielt werden können. Da Hybridisierungssonden jedoch sehr kostspielig sind, ist im Stand der Technik bislang kein Ansatz bzw. sind keine Hybridisierungszusammensetzungen für automatisierte In-Situ-Hybridisierungen bekannt, welcher bzw. welche auch unter Berücksichtigung ökonomischer Aspekte durchführbar ist.

Der vorliegenden Erfindung liegt somit die Aufgabe zugrunde, Zusammensetzungen bereitzustellen, welche zur Verwendung bei der In-Situ-Hybridisierung, insbesondere zur Detektion von Nukleinsäuren in einer biologischen Probe, geeignet sind und die zuvor geschilderten Nachteile des Standes der Technik zumindest weitgehend vermeiden oder aber wenigstens abschwächen.

Insbesondere liegt der vorliegenden Erfindung die Aufgabe zugrunde, Zusammensetzungen bzw. Hybridisierungslösungen für die In-Situ-Hybridisierung bereitzustellen, welche auch für den Einsatz in automatisierten In-Situ-Hybridisierungsverfahren geeignet sind bzw. eine kosteneffiziente Durchführung von automatisierten In-Situ-Hybridisierungsverfahren erlauben. Darüber hinaus liegt der vorliegenden Erfindung die Aufgabe zugrunde, Zusammensetzungen für die In-Situ-Hybridisierung bereitzustellen, welche sowohl bei kurzen als auch bei langen Hybridisierungs- bzw. Renaturierungsdauern zu guten Ergebnissen bzw. gut auswertbaren Signalmustern führen.

### Beschreibung

Zur Lösung der zuvor geschilderten Aufgabe schlägt die vorliegende Erfindung eine Zusammensetzung gemäß Anspruch 1 vor; weitere vorteilhafte Ausgestaltungen sind Gegenstand der diesbezüglich abhängigen Ansprüche.

Weiterhin ist Gegenstand der vorliegenden Erfindung die Verwendung einer Zusammensetzung nach der vorliegenden Erfindung gemäß dem diesbezüglichen unabhängigen Anspruch.

Wiederum weiterer Gegenstand der vorliegenden Erfindung ist ein Verfahren zur Detektion von Nukleinsäuren bzw. Chromosomenaberrationen gemäß dem diesbezüglichen unabhängigen Anspruch.

Schließlich ist Gegenstand der vorliegenden Erfindung ein Kit bzw. Kit-of-parts oder System zur Detektion von Nukleinsäuren bzw. Chromosomenaberrationen gemäß dem diesbezüglich unabhängigen Anspruch; weitere, vorteilhafte Eigenschaften sind Gegenstand des diesbezüglichen Unteranspruchs.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichts- oder volumenbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen, stets 100 % bzw. 100 Gew.-% resultieren. Dies versteht der Fachmann aber von selbst.

Im Übrigen gilt, dass der Fachmann anwendungsbezogen oder einzelfallbedingt von den nachfolgend angeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **ersten** erfindungsgemäßen Aspekt - eine Zusammensetzung, insbesondere Zusammensetzung zur Verwendung bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en),
wobei die Zusammensetzung enthält:
(a) mindestens eine Hybridisierungssonde ("Komponente (a)");
(b) mindestens ein polares protisches oder polares aprotisches Lösemittel ("Komponente (b)"), vorzugsweise mit cyclischer Molekülstruktur; und
(c) mindestens ein Carbonsäureamid und/oder dessen Salze ("Komponente (c)"), insbesondere Formamid und/oder dessen Salze, in einer Menge von mehr als 10 Vol.-%, bezogen auf die Zusammensetzung.

Mit anderen Worten ist es somit erfindungsgemäß vorgesehen, Zusammensetzungen für die Hybridisierung, synonym auch als Hybridisierungslösungen bezeichnet, bereitzustellen, in welcher bzw. welchen die Hybridisierungssonden auf Basis der Kombination mindestens eines zielgerichtet ausgewählten polaren protischen oder polaren aprotischen Lösemittels einerseits und mindestens eines Carbonsäureamids bzw. dessen Salzen, vorzugsweise Formamid, in einer Menge von mehr als 10 Gew.-%, bezogen auf die Zusammensetzung, stabilisiert bzw. gelöst vorliegen.

Denn im Rahmen der vorliegenden Erfindung wurde vollkommen überraschend gefunden, dass sich durch die zielgerichtete Kombination mindestens eines speziell ausgewählten polaren protischen oder polaren aprotischen Lösemittels einerseits und mindestens eines Carbonsäureamids bzw. dessen Salzen, insbesondere Formamid bzw. dessen Salzen, andererseits in jeweils definierten Mengen auch geringe Mengen an Hybridisierungssonden, d.h. stark verdünnte Hybridisierungssonden, in hochvolumigen Hybridisierungslösungen stabilisieren lassen. Mit diesen erfindungsgemäßen hochvolumigen Hybridisierungslösungen mit den Hybridisierungssonden in starker Verdünnung werden überraschend auch im Rahmen von automatischen In-Situ-Hybridisierungs-Verfahren hervorragende Signalmuster erhalten. Dies ist bislang im Stand der Technik nicht gelungen.

Weiterhin war es vollkommen überraschend, dass die erfindungsgemäßen Hybridisierungslösungen nicht nur für den Einsatz in automatisierten Hybridisierungsverfahren geeignet sind, sondern darüber hinaus sowohl bei kurzen als auch bei langen Hybridisierungs- bzw. Renaturierungsdauern zu hervorragenden Ergebnissen bzw. Signalmustern führen. Die erfindungsgemäßen Hybridisierungslösungen sind somit auch für den Einsatz in Schnell-Test, wie z.B. Fast-ISH bzw. Fast-FISH, oder sogenannten "flexiblen" In-Situ-Hybridisierungen mit variablen Hybridisierungszeiten geeignet.

Die vorliegende Erfindung ist insgesamt mit zahlreichen Vorteilen und Besonderheiten verbunden, welche nachfolgend in nichtbeschränkender Weise diskutiert sind und als Indiz für die Patentfähigkeit der vorliegenden Erfindung zu werten sind.

Wie zuvor geschildert, ist es im Rahmen der vorliegenden Erfindung überraschend gelungen, auch geringe Mengen an Hybridisierungssonden in hochvolumigen Hybridisierungslösungen bzw. -zusammensetzungen zu stabilisieren, so dass nunmehr auch automatische bzw. automatisierte In-Situ-Hybridisierungsverfahren unter Erhalt gut auswertbarer Signalmuster mit starken Signalen durchgeführt werden können, ohne dass im Vergleich zu manuellen bzw. etablierten Hybridisierungsverfahren größere Sondenmengen eingesetzt werden müssen.

Die erfindungsgemäßen Zusammensetzungen bzw. Hybridisierungslösungen sind somit insbesondere auch im Hinblick auf ökonomische Aspekte der Hybridisierungsverfahren vorteilhaft, da nunmehr die automatisierten Hybridisierungsverfahren als solche bereits kosteneffizient durchgeführt werden können. Darüber hinaus wird zudem die Verfahrenseffizienz von In-Situ-Hybridisierungen insgesamt gesteigert, da automatisierte Verfahren mit einem größeren Probendurchsatz verbunden sind.

Weiterhin sind die erfindungsgemäßen Hybridisierungslösungen vielseitig einsetzbar, da sie für verschiedene In-Situ-Hybridisierungs-Verfahren geeignet sind. Insbesondere können die erfindungsgemäßen Zusammensetzungen für Fluoreszenz-In-Situ-Hybridisierungen (FISH), Hellfeld-In-Situ-Hybridisierungen (BrISH), chromogene In-Situ-Hybridisierungen (CISH) und/oder Silber-In-Situ-Hybridisierungen (SISH) gleichermaßen eingesetzt werden.

Darüber hinaus eignen sich die Zusammensetzungen auch für In-Situ-Hybridisierungen sowohl mit langen als auch mit kurzen Hybridisierungs- bzw. Renaturierungsdauern. Die erfindungsgemäßen Zusammensetzungen führen insbesondere im Rahmen von Schnelltests mit kurzen Hybridisierungsdauern, wie Fast-ISH-Verfahren, oder flexiblen In-Situ-Hybridisierungen mit variablen Hybridisierungsdauern zu hervorragenden Ergebnissen. Dies ist insbesondere vor dem Hintergrund überraschend, als die erfindungsgemäßen Zusammensetzungen Formamid in Mengen von mindestens 10 Gew.-% enthalten und Formamid üblicherweise lange Hybridisierungszeiten erfordert. Die kurzen Hybridisierungsdauern trotz des Einsatzes von Formamid werden erst durch die erfindungsgemäße zielgerichtete Kombination des mindestens einen Carbonsäureamids, insbesondere des Formamids, mit mindestens einem polaren protischen oder polaren aprotischen Lösemittel in jeweils definierten Mengen ermöglicht.

Die erfindungsgemäßen Zusammensetzungen zeichnen sich insgesamt durch ihre hervorragende Stabilität, insbesondere der darin enthaltenen Hybridisierungssonden, aus. Die Stabilität der Zusammensetzungen kann noch weiterführend gesteigert werden, wenn die Zusammensetzungen Stabilisierungs- bzw. Blockiermittel, insbesondere auf Basis von Nukleinsäuren und/oder Nukleinsäureanaloga, enthalten, da diese einer vorzeitigen Degradation der Hybridisierungssonden vorbeugen. Überraschenderweise erlaubt die erfindungsgemäße Kombination von mindestens einem polaren protischen oder polaren aprotischen Lösemittel und Formamid, dass große Mengen an Stabilisierungs- bzw. Blockiermittel, insbesondere auf Basis von Nukleinsäuren, in den erfindungsgemäßen Zusammensetzungen gelöst werden können.

Schließlich zeichnen sich die mit den erfindungsgemäßen Zusammensetzungen erhaltenen Signalmuster der In-Situ-Hybridisierungen insgesamt durch ihre hervorragende Qualität aus. Es werden distinkte und gut auswertbare Signale erzeugt. Darüber hinaus können auch unspezifische Signale bzw. unspezifische Hintergrundfärbungen zumindest im Wesentlichen minimiert werden, d.h. die erfindungsgemäßen Hybridisierungszusammensetzungen zeichnen sich durch ihre hervorragende Stringenz aus.

Zum besseren Verständnis der vorliegenden Erfindung werden nachfolgend die zentralen Begrifflichkeiten und Bezeichnungen der erfindungsgemäßen Zusammensetzung definiert:
Die erfindungsgemäß eingesetzte In-Situ-Hybridisierung beruht auf der Hybridisierung bzw. Paarung komplementärer Basen von Nukleinsäure-Einzelsträngen, insbesondere DNA-Einzelsträngen, so dass spezifische Nukleinsäuresequenzen, d.h. die zu detektierenden Chromosomen- bzw. DNA-Bereiche, in einer Probe, wie einem Gewebe oder einem Zellpräparat, detektiert werden können. Im Rahmen der In-Situ-Hybridisierung werden direkt oder indirekt markierte, synthetisch hergestellte, insbesondere lokusspezifische, Hybridisierungssonden auf Basis von Nukleinsäuren mit Nukleinsäure-Einzelsträngen der Probe hybridisiert und anschließend detektiert.

Grundsätzlich kann die In-Situ-Hybridisierung zu verschiedenen Stadien des Zellzyklus der untersuchten Zellen bzw. Zellkerne stattfinden bzw. durchgeführt werden, wobei sich eine Durchführung in der Metaphase, wenn die Chromosomen im kondensierten Zustand vorliegen, oder in der Interphase, wenn die Chromosomen dekondensiert vorliegen, etabliert haben. Je nach Ziel bzw. Zweck der In-Situ-Hybridisierung ist eine Durchführung an kondensierten Chromosomen in der Metaphase nicht immer möglich, insbesondere beispielsweise bei der Untersuchung der Zellen solider Tumore auf Chromosomenaberrationen. In diesen Fällen wird die In-Situ-Hybridisierung an sich in der Interphase befindenden Zellen durchgeführt.

Die Hybridisierungssonden sind vorzugsweise lokusspezifisch. Unter lokusspezifischen Hybridisierungssonden werden im Rahmen der vorliegenden Erfindung für einen zu detektierenden Chromosomenbereich bzw. DNA-Bereich spezifische bzw. zu einem zu detektierenden Chromosomenbereich bzw. DNA-Bereich des DNA-Materials bzw. des genetischen Materials in einer zu untersuchenden Probe komplementäre Sonden verstanden. Auch kann es vorgesehen sein, die RNA vorzugsweise einzelner Gene zu detektieren bzw. lokusspezifische Hybridisierungssonden einzusetzen, welche für die RNA vorzugsweise einzelner Gene spezifisch sind. Üblicherweise basieren die erfindungsgemäß verwendeten Hybridisierungssonden auf Nukleinsäuren bzw. Nukleinsäurefragmenten und/oder Nukleinsäureanaloga und sind imstande, an den zu detektierenden Chromosomenbereich bzw. DNA-Bereich spezifisch zu binden bzw. zu hybridisieren. Bei den Nukleinsäuren und/oder Nukleinsäureanaloga kann es sich insbesondere um DNA, RNA, *locked nucleic acids* (LNA) oder um *peptide nucleic acids* (PNA) handeln. Zu Detektionszwecken sind die lokusspezifischen Hybridisierungssonden darüber hinaus direkt oder indirekt mit Detektionslabeln markiert. Der zu detektierende Chromosomenbereich bzw. DNA-Bereich kann eine variable Länge aufweisen. Insbesondere kann es vorgesehen sein, dass ein zu detektierender Chromosomenbereich bzw. DNA-Bereich bzw. eine zu detektierende RNA ein einziges bzw. ein einzelnes Gen teilweise oder vollständig umfasst. Gleichermaßen kann es auch vorgesehen sein, dass ein zu detektierender Chromosomenbereich bzw. DNA-Bereich mehrere Gene, vorzugsweise benachbarte Gene, bevorzugt zwei Gene, teilweise oder vollständig umfasst. Weiterhin kann es im Rahmen der vorliegenden Erfindung auch vorgesehen sein, dass es sich bei den eingesetzten Hybridisierungssonden um *Whole Chromosome Painting* (WCP)-Hybridisierungssonden oder *Partial Chromosome Painting* (PCP)-Sonden handelt, welche eine Vielzahl verschiedener lokusspezifischer Hybridisierungssonden umfassen, welche jeweils mit mindestens Detektionslabel markiert sind und die Detektion ganzer Chromosomen und/oder Chromosomenabschnitte erlauben. Was die Ausgestaltung der Hybridisierungssonden als solche anbelangt, so ist diese dem Fachmann geläufig, so dass es diesbezüglich keiner weiteren Informationen bedarf.

Was darüber hinaus den Begriff der biologischen Probe anbelangt, so kann es sich hierbei insbesondere um zu untersuchende Gewebe bzw. Gewebebestandteile, insbesondere um Gewebeschnitte, handeln. Darüber hinaus kann es jedoch auch vorgesehen sein, einzelne Zellen oder Zellaggregationen oder isolierte Zellkerne als biologische Probe einzusetzen.

Im Rahmen der vorliegenden Erfindung werden unter polaren aprotischen Lösemitteln insbesondere nichtwässrige Lösemittel verstanden, welche kein ionisierbares Proton im Molekül enthalten. Mit anderen Worten basieren aprotische Lösemittel insbesondere auf Molekülen, welche nicht über funktionelle Gruppen verfügen, aus welchen Wasserstoffatome in Form von Protonen abgegeben werden können bzw. dissoziieren. Darüber hinaus sind im Rahmen der vorliegenden Erfindung aprotisch polare Lösemittel vorzugsweise mit stark polarisierenden funktionellen Gruppen, wie Carbonylgruppen, Nitrilgruppen oder Thiolgruppen, substituiert, so dass die dem Lösemittel zugrundeliegenden Moleküle ein Dipolmoment aufweisen. Polare aprotische Lösemittel können sowohl in aliphatischer als auch aromatischer oder cyclischer Form vorliegen. Erfindungsgemäß hat es sich als besonders vorteilhaft erwiesen, polare aprotische Lösemittel mit cyclischer Molekülstruktur einzusetzen. Darüber hinaus ist es im Rahmen der vorliegenden Erfindung vorgesehen, dass die erfindungsgemäßen Zusammensetzungen kein Dimethylsulfoxid (DMSO) enthalten bzw. frei von DMSO sind. Für weitergehende Einzelheiten zu dem Begriff des polaren aprotischen Lösemittels kann verwiesen werden auf RÖMPP Chemielexikon, 10. Auflage, Thieme Verlag, Stuttgart New York, 1996, Seite 241, Stichwort: "Aprotische Lösungsmittel" sowie die darin referierte Literatur, welche hiermit vollumfänglich eingeschlossen ist.

Unter polaren protischen Lösemitteln hingegen werden im Rahmen der vorliegenden Erfindung insbesondere nichtwässrige Lösemittel verstanden, welche ein ionisierbares Proton im Molekül enthalten und/oder freizusetzen imstande sind und/oder Wasserstoff-Brückenbindungen auszubilden imstande sind. Mit anderen Worten basieren im Rahmen der vorliegenden Erfindung polare protische Lösemittel insbesondere auf Molekülen, welche über funktionelle Gruppen verfügen, aus welchen Wasserstoffatome in Form von Protonen abgegeben werden können bzw. dissoziieren, bzw. welche derart funktionalisiert sind, dass Wasserstoffatome in Form von Protonen abgegeben werden können bzw. dissoziieren können. Polare protische Lösemittel können darüber hinaus auch als polare amphiprotische Lösemittel bezeichnet werden. Für weitergehende Einzelheiten zu dem Begriff des polaren protischen Lösemittels kann verwiesen werden auf RÖMPP Chemielexikon, 10. Auflage, Thieme Verlag, Stuttgart New York, 1996, Seite 3597, Stichwort: "Protische Lösungsmittel" sowie die darin referierte Literatur, welche hiermit vollumfänglich eingeschlossen ist.

Unter Carbonsäureamiden werden im Rahmen der vorliegenden Erfindung Derivate des Ammoniaks sowie von primären und sekundären Aminen verstanden, bei welchen ein oder mehrere Wasserstoffatome des Stickstoffs durch Carbonsäurereste ersetzt bzw. durch Carbonsäurereste substituiert sind. Besonders gute Ergebnisse hinsichtlich der In-Situ-Hybridisierungen werden erzielt, wenn die erfindungsgemäßen Zusammensetzungen als Carbonsäureamid Formamid enthalten, bei welchem es sich um das Amid der Ameisensäure und somit um das einfachste Carbonsäureamid handelt. In einer besonders bevorzugten Ausführungsform ist das Carbonsäureamid daher Formamid. Was darüber hinaus die eingesetzte Menge an Carbonsäureamid, insbesondere Formamid, anbelangt, beträgt diese erfindungsgemäß mehr als 10 Vol.-%, d.h. eine Menge von genau 10 Vol.-% wird ausgeschlossen.

Die erfindungsgemäße Zusammensetzung kann in vielfältiger Art und Weise ausgestaltet sein. Bevorzugte Ausführungsformen sind nachfolgend im Detail geschildert.

Im Rahmen der vorliegenden Erfindung ist es überraschend gelungen, auch mit hochvolumigen Hybridisierungszusammensetzungen, welche die Hybridisierungssonden nur in geringen Konzentrationen und/oder Mengen bzw. auf das eingesetzte Volumen bezogen geringe Mengen an Hybridisierungssonden enthalten, gut auswertbare Signalmuster im Rahmen von In-Situ-Hybridisierungen zu erhalten.

Besonders gute Ergebnisse werden erfindungsgemäß erzielt, wenn die Zusammensetzung (a) die mindestens eine, vorzugsweise lokusspezifische, Hybridisierungssonde in einer Konzentration im Bereich von 0,1 ng/µl bis 50 ng/µl, insbesondere 0,5 ng/µl bis 50 ng/µl, vorzugsweise 0,7 ng/µl bis 8 ng/µl, bevorzugt 1 ng/µl bis 5 ng/µl, bezogen auf die Zusammensetzung, enthält. Im Rahmen der vorliegenden Erfindung war es vollkommen überraschend, dass die, vorzugsweise lokusspezifischen, Hybridisierungssonden in den vorgenannten Konzentrationen in den erfindungsgemäßen Hybridisierungslösungen stabilisiert werden können und so auch bei Einsatz in automatisierten Hybridisierungs-Verfahren zu hervorragenden Signalmustern führen. Die Bereitstellung von Hybridisierungslösungen, welche auch eine ökonomische bzw. kostensparende Durchführung von automatisierten Hybridisierungs-Verfahren erlauben, ist bislang im Stand der Technik nicht gelungen. Was das mindestens eine polare protische oder polare aprotische Lösemittel, vorzugsweise mit cyclischer Molekülstruktur, im Speziellen anbelangt, so ist dieses vorzugsweise ausgewählt aus der Gruppe von Lösemitteln mit Lacton-, Sulfon-, Nitril-, Carbonat- und/oder Amid-Funktionalität, stärker bevorzugt aus der Gruppe von Lösemitteln mit Lacton-, Sulfon-, Carbonat- und/oder Amid-Funktionalität. Der Begriff "Funktionalität" beschreibt in diesem Zusammenhang das Vorhandensein funktioneller Gruppen in einem Molekül. Mit anderen Worten, das polare protische oder polare aprotische Lösemittel ist vorzugsweise ausgewählt aus der Gruppe bestehend aus Lösemitteln, die mindestens eine funktionelle Lacton-, Sulfon-, Nitril-, Carbonat- und/oder Amidgruppe aufweisen.

Insbesondere kann es vorgesehen sein, dass das mindestens eine polare aprotische oder polare protische Lösemittel ausgewählt ist aus der Gruppe von Ethylencarbonat, Pyrrolidonen, Lactamen, Ethylensulfit, γ-Butyrolacton, Ethylentrithiocarbonat, Propylencarbonat und/oder Sulfolan, insbesondere bevorzugt Ethylencarbonat und/oder Pyrrolidonen, stärker bevorzugt Ethylencarbonat.

Auch kann es im Rahmen der vorliegenden Erfindung vorgesehen sein, dass das mindestens eine Lösemittel, vorzugsweise mit cyclischer Molekülstruktur, ein polares aprotisches Lösemittel ist, insbesondere ausgewählt aus cyclischen Carbonaten (cyclischen Kohlensäurestern), insbesondere cyclischen Kohlensäureestern von Alkylenglykolen, bevorzugt Ethylencarbonat (1,3-Dioxolan-2-on) und Propylencarbonat, besonders bevorzugt Ethylencarbonat, sowie cyclischen Mono-, Di- und Trithiocarbonaten, insbesondere Ethylensulfit und Ethylentrithiocarbonat.

Weiterhin werden erfindungsgemäß gute Ergebnisse erzielt, wenn das mindestens eine Lösemittel, vorzugsweise mit cyclischer Molekülstruktur, ein polares aprotisches Lösemittel ist, insbesondere ausgewählt aus aprotischen cyclischen Amiden (Lactamen), insbesondere N-alkylsubstituierten Pyrrolidonen, vorzugsweise N-Methyl-2-pyrrolidon und/oder N-Ethyl-2-pyrrolidon.

Auch kann es erfindungsgemäß vorgesehen sein, dass das mindestens eine Lösemittel, vorzugsweise mit cyclischer Molekülstruktur, ein polares protisches Lösemittel ist, insbesondere ausgewählt aus protischen cyclischen Amiden (Lactamen), insbesondere protischen cyclischen Amiden (Lactamen) mit einem Wasserstoffatom am Amid-Stickstoff (Lactam-Stickstoff), bevorzugt ausgewählt aus der Gruppe von 2-Pyrrolidon (γ-Butyrolactam), 3-Pyrrolidon, Caprolactamen und/oder 2-Piperidon (Valerolactam), besonders bevorzugt Pyrrolidonen, ganz besonders bevorzugt 2-Pyrrolidon (γ-Butyrolactam).

Das mindestens eine polare protische oder polare aprotische Lösemittel weist vorzugsweise eine cyclische Molekülstruktur auf. Bevorzugt sind cyclische Molekülstrukturen mit einem aus fünf oder sechs Ringatomen bestehenden Ringsystem. Die cyclische Molekülstruktur kann ein Homo- oder Heterocyclus sein, vorzugsweise ein Heterozyklus. Ein Heterocyclus ist eine Verbindung mit ringbildenden Atomen aus mindestens zwei verschiedenen Elementen. Vorzugsweise umfasst der Heterozyklus neben ringbildenden Kohlenstoffatomen auch ein oder mehrere ringbildende Stickstoff-, Schwefel- und/oder Sauerstoffatome, bevorzugt ein oder mehrere Stickstoffatome (N-Heterozyklus) und/oder ein oder mehrere Sauerstoffatome. Darüber hinaus kann es im Rahmen der vorliegenden Erfindung vorgesehen sein, dass das mindestens eine Lösemittel ein polares protisches Lösemittel mit cyclischer Molekülstruktur ist, wobei die cyclische Molekülstruktur einen N-Heterocyclus mit freiem Wasserstoffatom am Stickstoffatom umfasst.

Das mindestens eine polare protische oder polare aprotische Lösemittel weist vorzugsweise eine cyclische Molekülstruktur auf, wobei die cyclische Molekülstruktur einen Heterozyklus umfasst, vorzugsweise einen Heterozyklus mit einem oder mehreren ringbildenden Stickstoff-, Schwefel- und/oder Sauerstoffatomen.

Vorzugsweise weist die cyclische Molekülstruktur Lacton-, Sulfon-, Nitril-, Carbonat- und/oder Amid-Funktionalität auf, stärker bevorzugt Lacton-, Sulfon-, Carbonat- und/oder Amid-Funktionalität. In einer bevorzugten Ausführungsform betrifft die Erfindung somit eine Zusammensetzung, die umfasst oder besteht aus: (a) mindestens einer Hybridisierungssonde ("Komponente (a)"); (b) mindestens einem polaren protischen oder polaren aprotischen Lösungsmittel mit cyclischer Molekülstruktur, wobei die cyclische Molekülstruktur Lacton-, Sulfon-, Carbonat- und/oder Amid-Funktionalität aufweist ("Komponente (b)"); und mindestens einem Carbonsäureamid und/oder dessen Salzen ("Komponente (c)") in einer Menge von mehr als 10 Vol.-% bezogen auf die Zusammensetzung.

Gemäß einer besonders bevorzugten Ausführungsform der vorliegenden Erfindung kann es vorgesehen sein, dass das mindestens eine polare protische oder polare aprotische Lösemittel ausgewählt ist aus der Gruppe von Ethylencarbonat, 2-Piperidon (Valerolactam), 2-Pyrrolidon (γ-Butyrolactam), 3-Sulfolen ("Butdiene Sulfone") und/oder γ-Butyrolacton. Die besten Ergebnisse werden erfindungsgemäß erzielt, wenn das polare protische oder polare aprotische Lösemittel γ-Butyrolacton, 2-Pyrrolidon (γ-Butyroiactam) und/oder Ethylencarbonat ist. In diesem Zusammenhang wird auch auf die seitens der Anmelderin durchgeführten und nachfolgend geschilderten Ausführungsbeispiele verwiesen, welche die überlegenen Eigenschaften der zuvor genannten polaren protischen oder polaren aprotischen Lösemittel in den erfindungsgemäßen Zusammensetzungen belegen.

Durch den Einsatz mindestens eines polaren protischen oder polaren aprotischen Lösemittels in der erfindungsgemäßen Zusammensetzung - insbesondere in Kombination mit mindestens einem Carbonsäureamid, vorzugsweise Formamid - können einerseits die mit dem Einsatz von Formamid einhergehenden Nachteile in Hybridisierungslösungen, wie lange Hybridisierungsdauern, kompensiert werden. Darüber hinaus wird - ohne sich hierbei auf diese Theorie beschränken zu wollen - durch den Einsatz von polaren protischen oder aprotischen Lösemitteln in den erfindungsgemäßen Zusammensetzungen die Löslichkeit für Nukleinsäuren insgesamt erhöht, was wiederum die Stabilität der erfindungsgemäßen Hybridisierungszusammensetzungen verbessert. Denn durch die gute Löslichkeitskapazität für Nukleinsäuren können große Mengen an nichthybridisierenden, unspezifischen Nukleinsäuren, insbesondere Stabilisierungs-DNA, als sogenannte Blockier- bzw. Stabilisierungsmittel in die Zusammensetzungen eingebracht werden, welche einerseits einer Degradation der, vorzugsweise lokusspezifischen, Hybridisierungssonden vorbeugen und darüber hinaus das Signalmuster insgesamt verbessern und Signalen durch unspezifische Bindungen bzw. Hybridisierungen vorbeugen, d.h. auch zu einer Erhöhung der Stringenz führen.

Was die eingesetzte Menge des mindestens einen polaren protischen oder polaren aprotischen Lösemittels anbelangt, so kann diese in weiten Bereichen variieren. Erfindungsgemäß kann es insbesondere vorgesehen sein, dass die Zusammensetzung (b) das mindestens eine polare protische oder polare aprotische Lösemittel, vorzugsweise mit cyclischer Molekülstruktur, in einer Menge enthält, welche nicht zur Denaturierung von Nukleinsäuren führt. Im Rahmen der vorliegenden Erfindung ist es insbesondere vorgesehen, durch das mindestens eine polare protische oder polare aprotische Lösemittel die Nukleinsäuren in der erfindungsgemäßen Zusammensetzung zu lösen und zu stabilisieren, wobei das Lösemittel jedoch in einer Menge eingesetzt wird, welche alleine, d.h. ohne den zusätzlichen Einsatz mindestens eines Carbonsäureamids und/oder weiterer denaturierender Mittel, nicht zur Denaturierung der Nukleinsäuren führen würde.

Insbesondere kann die Zusammensetzung nach der vorliegenden Erfindung das mindestens eine polare protische oder polare aprotische Lösemittel, vorzugsweise mit cyclischer Molekülstruktur, in einer Menge im Bereich von 0,5 bis 40 Vol.-% oder Gew.-%, insbesondere 1 bis 35 Vol.-% oder Gew.-%, vorzugsweise 2 bis 30 Vol.-% oder Gew.-%, bevorzugt 5 bis 20 Vol.-% oder Gew.-%, besonders bevorzugt 7 bis 15 Vol.-% oder Gew.-%, bezogen auf die Zusammensetzung, enthalten.

Darüber hinaus ist es im Rahmen der vorliegenden Erfindung bevorzugt, wenn die Zusammensetzung (b) das mindestens eine polare protische oder polare aprotische Lösemittel, vorzugsweise mit cyclischer Molekülstruktur, in einer Menge von mindestens 0,5 Vol.-% oder Gew.-%, insbesondere mindestens 1 Vol.-% oder Gew.-%, vorzugsweise mindestens 2 Vol.-% oder Gew.-%, bevorzugt mindestens 5 Vol.-% oder Gew.-%, besonders bevorzugt mindestens 7 Vol.-% oder Gew.-%, ganz besonders bevorzugt mindestens 10 Vol.-% oder Gew.-%, enthält. Gleichermaßen kann es vorgesehen sein, dass die Zusammensetzung (b) das mindestens eine polare protische oder polare aprotische Lösemittel, vorzugsweise mit cyclischer Molekülstruktur, in einer Menge von höchstens 50 Vol.-% oder Gew.-%, insbesondere höchstens 40 Vol.-% oder Gew.-%, vorzugsweise höchstens 30 Vol.-% oder Gew.-%, bevorzugt höchstens 20 Vol.-% oder Gew.-%, besonders bevorzugt höchstens 15 Vol.-% oder Gew.-%, ganz besonders bevorzugt höchstens 13 Vol.-% oder Gew.-%, bezogen auf die Zusammensetzung, enthält.

Die vorstehenden Prozentangaben bezüglich des mindestens einen polaren protischen oder polaren aprotischen Lösemittels sind vorzugsweise Gew.-%-Angaben. Anders gesagt, enthält die Zusammensetzung nach der vorliegenden Erfindung das mindestens eine polare protische oder polare aprotische Lösemittel, vorzugsweise mit cyclischer Molekülstruktur, vorzugsweise in einer Menge im Bereich von 0,5 bis 40 Gew.-%, insbesondere 1 bis 35 Gew.-%, vorzugsweise 2 bis 30 Gew.-%, bevorzugt 5 bis 20 Gew.-%, besonders bevorzugt 7 bis 15 Gew.-%, bezogen auf die Zusammensetzung.

Im Rahmen der vorliegenden Erfindung hat sich insgesamt gezeigt, dass die Einhaltung der vorgenannten Mengenbereiche die erhaltenen Signalmuster signifikant verbessert. Auch die Menge des mindestens einen Carbonsäureamids bzw. von dessen Salzen, insbesondere des Formamids bzw. dessen Salzen, hat sich als kritischer Faktor in Bezug auf die Signalstärken bzw. Signalmuster erwiesen. Im Zusammenhang mit den Mengen des mindestens einen polaren protischen oder polaren aprotischen Lösemittels einerseits und des mindestens einen Carbonsäureamids, insbesondere des Formamids, andererseits wird auch auf die seitens der Anmelderin durchgeführten und nachfolgend im Detail geschilderten Ausführungsbeispiele verwiesen, welche belegen, dass erst der Einsatz speziell ausgewählter Mengen von Lösemittel und Carbonsäureamid zu den hervorragenden Signalmustern bei der Durchführung von In-Situ-Hybridisierungen, insbesondere auf einem Automaten bzw. als Schnelltest mit kurzen bzw. flexiblen Hybridisierungsdauern, führt.

Im Rahmen der vorliegenden Erfindung kann es insbesondere vorgesehen sein, dass die Zusammensetzung (c) das mindestens eine Carbonsäureamid und/oder dessen Salze, insbesondere Formamid und/oder dessen Salze, in einer Menge enthält, welche zur Denaturierung von Nukleinsäuren führt. In einer bevorzugten Ausführungsform enthält die Zusammensetzung (c) Formamid und/oder dessen Salze in einer Menge, welche zur Denaturierung von Nukleinsäuren führt bzw. dazu geeignet ist. Mit anderen Worten kann es somit im Rahmen der vorliegenden Erfindung vorgesehen sein, dass die Zusammensetzung das mindestens eine Carbonsäureamid, insbesondere Formamid, in einer Menge enthält, welche allein, d.h. ohne polares protisches oder polares aprotisches Lösemittel, bereits zur Denaturierung der Nukleinsäuren führen würde.

Was die eingesetzten Mengen des mindestens einen Carbonsäureamids weiterhin anbelangt, so hat es sich als vorteilhaft erwiesen, wenn die Zusammensetzung (c) das mindestens eine Carbonsäureamid und/oder dessen Salze, insbesondere Formamid und/oder dessen Salze, in einer Menge im Bereich von 10 bis 60 Vol.-%, insbesondere 15 bis 50 Vol.-%, vorzugsweise 17 bis 40 Vol.-%, bevorzugt 20 bis 30 Vol.-%, bezogen auf die Zusammensetzung, enthält. In einer bevorzugten Ausführungsform enthält die erfindungsgemäße Zusammensetzung das mindestens eine Carbonsäureamid und/oder dessen Salze, insbesondere Formamid und/oder dessen Salze, in einer Menge im Bereich von 17 und 40 Vol.-%. In einer noch stärker bevorzugten Ausführungsform enthält die erfindungsgemäße Zusammensetzung das mindestens eine Carbonsäureamid und/oder dessen Salze, insbesondere Formamid und/oder dessen Salze, in einer Menge im Bereich von 20 und 30 Vol.-%.

Darüber hinaus kann es vorgesehen sein, dass die Zusammensetzung (c) das mindestens eine Carbonsäureamid und/oder dessen Salze, insbesondere Formamid und/oder dessen Salze, in einer Menge von mindestens 10 Vol.-%, insbesondere mindestens 15 Vol.-%, vorzugsweise mindestens 17 Vol.-%, bevorzugt mindestens 20 Vol.-%, bezogen auf die Zusammensetzung, enthält. Gleichermaßen kann es vorgesehen sein, dass die Zusammensetzung (c) das mindestens eine Carbonsäureamid und/oder dessen Salze, insbesondere Formamid und/oder dessen Salze, in einer Menge von höchstens 60 Vol.-%, insbesondere höchstens 50 Vol.-%, vorzugsweise höchstens 40 Vol.-%, bevorzugt höchstens 30 Vol.-%, bezogen auf die Zusammensetzung, enthält. In einer bevorzugten Ausführungsform enthält die erfindungsgemäße Zusammensetzung das mindestens eine Carbonsäureamid und/oder dessen Salze, insbesondere Formamid und/oder dessen Salze, in einer Menge von mindestens 17 Vol.-% bezogen auf die Zusammensetzung. In einer noch stärker bevorzugten Ausführungsform enthält die erfindungsgemäße Zusammensetzung das mindestens eine Carbonsäureamid und/oder dessen Salze, insbesondere Formamid und/oder dessen Salze, in einer Menge von mindestens 20 Vol.-% bezogen auf die Zusammensetzung.

Wie zuvor erwähnt, haben sich Mengen des Carbonsäureamids und/oder dessen Salzen von mehr als 10% besonders bewährt. Somit ist es erfindungsgemäß besonders bevorzugt, dass Zusammensetzung (c) das mindestens eine Carbonsäureamid und/oder dessen Salze, insbesondere Formamid und/oder dessen Salze, in einer Menge zwischen 10 und 60 Vol.-%, insbesondere zwischen 15 und 50 Vol.-%, vorzugsweise zwischen 17 und 40 Vol.-%, bevorzugt zwischen 20 und 30 Vol.-%, bezogen auf die Zusammensetzung, enthält. In einer besonders bevorzugten Ausführungsform enthält die Zusammensetzung (c) Formamid und/oder dessen Salze in einer Menge zwischen 17 und 40 Vol.-%.

Darüber hinaus ist es besonders bevorzugt, dass die Zusammensetzung (c) das mindestens eine Carbonsäureamid und/oder dessen Salze, insbesondere Formamid und/oder dessen Salze, in einer Menge von mehr als 10 Vol.-%, insbesondere mehr als 15 Vol.-%, vorzugsweise mehr als 17 Vol.-%, bevorzugt mehr als 20 Vol.-%, bezogen auf die Zusammensetzung, enthält. In einer besonders bevorzugten Ausführungsform enthält die Zusammensetzung (c) Formamid und/oder dessen Salze in einer Menge von mehr als 17 Vol.-%.

In einer Ausführungsform enthält die erfindungsgemäße Zusammensetzung mehr als 10 Vol.-% des Carbonsäureamids und/oder dessen Salze, bevorzugt Formamid und/oder dessen Salze. In einer stärker bevorzugten Ausführungsform enthält die erfindungsgemäße Zusammensetzung mehr als 20 Vol.-% des Carbonsäureamids und/oder dessen Salze, bevorzugt Formamid und/oder dessen Salze. Neben den eingesetzten absoluten Mengen hat es sich im Hinblick auf die mit der erfindungsgemäßen Zusammensetzung erhaltenen Signalmuster als vorteilhaft erwiesen, wenn das mindestens eine polare protische oder polare aprotische Lösemittel einerseits und das mindestens eine Carbonsäureamid, insbesondere Formamid, in definiertem Mengenverhältnis zueinander eingesetzt werden:
Besonders gute Ergebnisse werden im Rahmen der In-Situ-Hybridisierungen erzielt, wenn die Zusammensetzung die Komponente (b) und die Komponente (c) bzw. das mindestens eine polare protische oder polare aprotische Lösemittel, vorzugsweise mit cyclischer Molekülstruktur, und das mindestens eine Carbonsäureamid und/oder dessen Salze, insbesondere Formamid und/oder dessen Salze, in einem volumenbezogenen Verhältnis im Bereich von 1 : 100 bis 50 : 1, insbesondere 1 : 50 bis 20 : 1, vorzugsweise 1 : 25 bis 10 : 1, bevorzugt 1 : 10 bis 5 : 1, besonders bevorzugt 1 : 5 bis 1 : 1, ganz besonders bevorzugt 1 : 3 bis 1 : 2 , enthält.

Darüber hinaus hat es sich als vorteilhaft erwiesen, wenn die Zusammensetzung mindestens ein Polysaccharid ("Komponente (d)"), insbesondere Biopolysaccharid, vorzugsweise neutrales Biopolysaccharid, bevorzugt Dextran und/oder dessen Derivate oder Salze, besonders bevorzugt Dextransulfat, enthält. Im Rahmen der vorliegenden Erfindung hat sich überraschend gezeigt, dass die Hybridisierungsergebnisse mit den erfindungsgemäßen Zusammensetzungen noch weiterführend verbessert werden können, wenn die erfindungsgemäßen Zusammensetzungen mindestens ein Polysaccharid insbesondere zur Stabilisierung enthalten. Diesbezüglich wird auch auf die erfindungsgemäßen Ausführungsbeispiele verwiesen, welche den Effekt des Polysaccharids in den Zusammensetzungen belegen. Besonders gute Ergebnisse werden erzielt, wenn als Polysaccharid Dextransulfat eingesetzt wird.

Was die Menge des mindestens einen Polysaccharids in den Zusammensetzungen anbelangt, so hat es sich als besonders vorteilhaft erwiesen, wenn die Zusammensetzung die Komponente (d) in einer Menge im Bereich von 0,1 bis 50 Gew.-%, insbesondere 1 bis 40 Gew.-%, vorzugsweise 5 bis 30 Gew.-%, bevorzugt 10 bis 20 Gew.-%, bezogen auf die Zusammensetzung, enthält. In einer bevorzugten Ausführungsform enthält die Zusammensetzung von 5 bis 30 Gew.-% des mindestens einen Polysaccharids, besonders bevorzugt von 5 bis 30 Gew.-% Dextransulfat.

Darüber hinaus kann es im Rahmen der vorliegenden Erfindung vorgesehen sein, dass die Zusammensetzung mindestens ein chemisches Puffersystem, insbesondere in Form von Puffersalz(en) ("Komponente (e)"), enthält. Insbesondere dient das chemische Puffersystem zur Einstellung bzw. Konstanthaltung des pH-Werts der Zusammensetzung.

Die eingesetzten Mengen des chemischen Puffersystems können in weiten Bereichen variieren. Im Rahmen der vorliegenden Erfindung hat es sich als vorteilhaft erwiesen, wenn die Zusammensetzung (e) das chemische Puffersystem, bezogen auf die Zusammensetzung und berechnet als Summe aller Bestandteile des chemischen Puffersystems, in einer Menge im Bereich von 0,001 bis 5 Gew.-%, insbesondere 0,01 bis 4 Gew.-%, vorzugsweise 0,1 bis 3 Gew.-%, bevorzugt 0,5 bis 2 Gew.-%, besonders bevorzugt 1 bis 1,5 Gew.-%, enthält.

Was die Auswahl des Puffersystems anbelangt, so liegt diese im üblichen Können des Fachmanns. Besonders gute Ergebnisse werden im Rahmen der vorliegenden Erfindung erzielt, wenn das chemische Puffersystem mindestens ein Salz enthält, insbesondere mindestens ein Carbonsäuresalz, vorzugsweise ein Citrat, und/oder mindestens ein anorganisches Salz, insbesondere mindestens ein Alkali- und/oder Erdalkali-Salz, vorzugsweise mindestens ein Alkali- und/oder Erdalkali-Chlorid, besonders bevorzugt Natriumchlorid. Besonders bevorzugt ist das chemische Puffersystem ein citratbasiertes Puffersystem bzw. ein citratbasiertes Puffersystem auf Grundlage von tri-Natriumcitrat/Natriumchlorid.

Gemäß einer bevorzugten Ausführungsform wird das dem Fachmann bekannte chemische Puffersystem, z. B. ein SSC (saline-sodium citrate)-Puffer-System, auf Basis von triNatriumcitrat (0,3 M bei 20fach konzentriertem SSC) und Natriumchlorid (3 M bei 20fach konzentriertem SSC) eingesetzt. Darüber hinaus ist es auch möglich, andere, dem Fachmann als solche wohlbekannte Puffer-Systeme einzusetzen, wie beispielsweise HEPES [2-(4-(2-Hydroxyethyl)1-piperazinyl)-ethan-sulfonsäure], SSPE [sodium chloride / sodium phosphate / EDTA], PIPES [Piperazine-N,N'-bis(2-ethansulfonsäure)], TMAC [Tetramethylammoniumchlorid], TRIS [Tris(hydroxymethyl)-aminomethan] oder SET-Puffer.

Besonders gute Ergebnisse im Rahmen der vorliegenden Erfindung werden zudem erzielt, wenn die Zusammensetzung einen pH-Wert im Bereich von 5,0 bis 9,0, insbesondere im Bereich von 5,5 bis 8,5, vorzugsweise im Bereich von 6,0 bis 8,0, bevorzugt im Bereich von 6,5 bis 7,5, aufweist. Das erfindungsgemäße chemische Puffersystem ist insbesondere geeignet, diesen pH-Wert einzustellen und während der Lagerung und der Hybridisierungsreaktion zu halten.

Gemäß einer bevorzugten Ausführungsform der vorliegenden Erfindung kann es zudem vorgesehen sein, dass die Zusammensetzung mindestens ein Blockier- und/oder Stabilisierungsmittel ("Komponente (f)") enthält, insbesondere wobei das Blockier- und/oder Stabilisierungsmittel auf Nukleinsäuren und/oder Nukleinsäureanaloga basiert, vorzugsweise auf DNA und/oder RNA. Bei den Nukleinsäuren und/oder Nukleinsäureanaloga als Blockier- und/oder Stabilisierungsmittel kann es sich insbesondere auch um locked nucleic acids (LNA) oder um *peptide nucleic acids* (PNA) handeln. Der Einsatz mindestens eines Blockier- bzw. Stabilisierungsmittels ist in mehrfacher Hinsicht von Vorteil, da einerseits die eingesetzten Hybridisierungssonden stabilisiert werden können und einem vorzeitigen Abbau bzw. einer vorzeitigen Degradation der Sonden vorgebeugt werden kann. Andererseits können unspezifische Hintergrundsignale in dem Signalmuster der In-Situ-Hybridisierung minimiert werden.

Aufgrund der erfindungsgemäß eingesetzten Komponenten, insbesondere aufgrund der erfindungsgemäßen Kombination des mindestens einen polaren protischen oder polaren aprotischen Lösemittels einerseits und des mindestens einen Carbonsäureamids, insbesondere des Formamids, andererseits in jeweils definierten Mengen ist es möglich, große Mengen an Blockier- und/oder Stabilisierungsmittel(n) in den erfindungsgemäßen Zusammensetzung zu lösen. Somit kann es erfindungsgemäß vorgesehen sein, dass die Zusammensetzung (f) das mindestens eine Blockier- und/oder Stabilisierungsmittel in einer Konzentration im Bereich von 0,001 µg/µl bis 100 µg/µl, insbesondere 0,005 µg/µl bis 80 µg/µl, vorzugsweise 0,01 µg/µl bis 40 µg/µl, bevorzugt 0,05 µg/µl bis 20 µg/µl, besonders bevorzugt 0,1 µg/µl bis 10 µg/µl, bezogen auf die Zusammensetzung, enthält.

Darüber hinaus ist es im Hinblick auf die erfindungsgemäßen Zusammensetzungen von Vorteil, wenn die Zusammensetzung mindestens ein anorganisches Salz ("Komponente (g)"), insbesondere Alkali- und/oder Erdalkali-Salz, vorzugsweise Alkali- und/oder Erdalkali-Chlorid, besonders bevorzugt Natriumchlorid, enthält.

Die eingesetzten Mengen des mindestens einen anorganischen Salzes können in weiten Bereichen variieren. Besonders gute Ergebnisse werden mit der erfindungsgemäßen Zusammensetzung erzielt, wenn die Zusammensetzung (g) das mindestens eine anorganische Salz in einer Menge im Bereich von 0,01 bis 15 Gew.-%, insbesondere 0,05 bis 10 Gew.-%, vorzugsweise 0,1 bis 10 Gew.-%, bevorzugt 0,5 bis 5 Gew.-%, besonders bevorzugt 1 bis 3 Gew.-%, bezogen auf die Zusammensetzung, enthält.

Darüber hinaus hat es sich überraschend gezeigt, dass die im Rahmen der In-Situ-Hybridisierungen erhaltenen Signalmuster weiterführend verbessert werden können, wenn die erfindungsgemäßen Zusammensetzungen bzw. Hybridisierungslösungen mindestens ein Detergens und/oder Tensid ("Komponente (h)") enthalten. Unter einem Detergens bzw. Tensid werden im Rahmen der vorliegenden Erfindung Substanzen verstanden, welche imstande sind, die Oberflächenspannung von Flüssigkeiten bzw. die Grenzflächenspannung zwischen zwei Phasen herabzusetzen. Auf dieser Basis wird die Bildung von Dispersionen bzw. Lösungen gefördert bzw. verbessert.

Durch den Einsatz mindestens eines Detergens bzw. Tensids in den erfindungsgemäßen Zusammensetzungen bzw. Hybridisierungslösungen wird - ohne sich hierbei auf diese Theorie beschränken zu wollen - einerseits die spezifische Hybridisierung von Nukleinsäuren in der Probe und Hybridisierungssonden verbessert bzw. verstärkt. Andererseits wird - ebenfalls ohne sich hierbei auf diese Theorie beschränken zu wollen - die im Rahmen der Auswertung bzw. Analyse von In-Situ-Hybridisierungs-Proben bzw. -Präparaten üblicherweise durchgeführte Kernfärbung bzw. Färbung des Nukleus abgeschwächt, was wiederum zu einem verbesserten Kontrast von Signalen zu Hintergrund und insgesamt zu stärkeren Signalstärken führt.

Besonders gute Ergebnisse werden im Rahmen der vorliegenden Erfindung erzielt, wenn das Detergens und/oder Tensid ausgewählt ist aus nichtionischen Tensiden, vorzugsweise Polyalkylenglycolethern, besonders bevorzugt Polyalkylenglycolethern des Laurylalkohols und/oder des Cetylalkohols und/oder des Cetylstearylalkohols und/oder des Oleylalkohols, insbesondere des Laurylalkohols. In diesem Zusammenhang ist es besonders bevorzugt, wenn das Detergens und/oder Tensid ausgewählt ist aus Polyoxyethylen(4)laurylether, Polyoxyethylen(9)laurylether und/oder Polyoxyethylen(23)laurylether, insbesondere Polyoxyethylen(23)laurylether.

Die Mengen des eingesetzten Detergens bzw. Tensids sind variabel. Es hat sich als besonders vorteilhaft erwiesen, wenn die Zusammensetzung das mindestens eine Detergens und/oder Tensid in einer Menge im Bereich von 0,001 bis 5 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,12 bis 1 Gew.-%, bezogen auf die Zusammensetzung, aufweist.

Im Hinblick auf die vorteilhaften Eigenschaften von Detergenzien bzw. Tensiden wird auch die erfindungsgemäßen Ausführungsbeispiele verwiesen, welche deren positive Effekte auf die Hybridisierungsergebnisse bzw. Signalstärken belegen.

Was die Ausgestaltung der erfindungsgemäßen Zusammensetzung zudem anbelangt, kann es insbesondere vorgesehen sein, dass es sich um eine wässrige Lösung handelt. In diesem Zusammenhang ist es vorteilhaft, wenn die Zusammensetzung Wasser, insbesondere gereinigtes Wasser, in einer Menge im Bereich von 10 bis 99 Gew.-%, insbesondere im Bereich von 20 bis 95 Gew.-%, bevorzugt im Bereich von 30 bis 90 Gew.-%, besonders bevorzugt im Bereich von 40 bis 85 Gew.-%, bezogen auf die Zusammensetzung, enthält.

Zudem kann es im Rahmen der vorliegenden Erfindung vorgesehen sein, dass die Zusammensetzung Wasser in einer Menge enthält, dass in der Summe unter Einbeziehung aller Komponenten stets 100 % bzw. 100 Gew.-% resultieren, bezogen auf die Zusammensetzung.

Weiterhin kann es auch vorgesehen sein, dass die Zusammensetzung Wasser als Träger bzw. Exzipienten enthält. Gleichermaßen kann es vorgesehen sein, dass die Zusammensetzung wässrig ausgebildet ist.

Gegenstand der vorliegenden Erfindung - gemäß einer besonders bevorzugten Ausführungsform - ist somit eine Zusammensetzung zur Verwendung bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en), insbesondere eine Zusammensetzung, wie sie zuvor beschrieben wurde,
wobei die Zusammensetzung enthält:
(a) mindestens eine, vorzugsweise lokusspezifische, Hybridisierungssonde ("Komponente (a)"), insbesondere in einer Konzentration im Bereich von 0,1 ng/µl bis 50 ng/µl, insbesondere 0,5 ng/µl bis 50 ng/µl, vorzugsweise 0,7 ng/µl bis 8 ng/µl, bevorzugt 1 ng/µl bis 5 ng/µl, bezogen auf die Zusammensetzung;
(b) mindestens ein polares protisches oder polares aprotisches Lösemittel ("Komponente (b)"), vorzugsweise mit cyclischer Molekülstruktur, insbesondere in einer Menge im Bereich von 0,5 bis 40 Vol.-%, insbesondere 1 bis 35 Vol.-%, vorzugsweise 2 bis 30 Vol.-%, bevorzugt 5 bis 20 Vol.-%, besonders bevorzugt 7 bis 15 Vol.-%, bezogen auf die Zusammensetzung;
(c) mindestens ein Carbonsäureamid und/oder dessen Salze ("Komponente (c)"), insbesondere Formamid und/oder dessen Salze, insbesondere in einer Menge im Bereich von 10 bis 60 Vol.-%, insbesondere 15 bis 50 Vol.-%, vorzugsweise 17 bis 40 Vol.-%, bevorzugt 20 bis 30 Vol.-%, bezogen auf die Zusammensetzung;
(d) gegebenenfalls mindestens ein Polysaccharid ("Komponente (d)"), vorzugsweise neutrales Biopolysaccharid, bevorzugt Dextran und/oder dessen Derivate oder Salze, insbesondere in einer Menge im Bereich von 0,1 bis 50 Gew.-%, insbesondere 1 bis 40 Gew.-%, vorzugsweise 5 bis 30 Gew.-%, bevorzugt 10 bis 20 Gew.-%, besonders bevorzugt 13 bis 18 Gew.-%, bezogen auf die Zusammensetzung;
(e) gegebenenfalls mindestens ein chemisches Puffersystem ("Komponente (e)"), insbesondere in Form von Puffersalz(en), insbesondere in einer Menge im Bereich von 0,001 bis 5 Gew.-%, insbesondere 0,01 bis 4 Gew.-%, vorzugsweise 0,1 bis 3 Gew.-%, bevorzugt 0,5 bis 2 Gew.-%, besonders bevorzugt 1 bis 1,5 Gew.-%, bezogen auf die Zusammensetzung und berechnet als Summe aller Bestandteile des chemischen Puffersystems;
(f) gegebenenfalls mindestens ein Blockier- und/oder Stabilisierungsmittel ("Komponente (f)"), insbesondere in einer Konzentration im Bereich von 0,001 µg/µl bis 100 µg/µl, insbesondere 0,005 µg/µl bis 80 µg/µl, vorzugsweise 0,01 µg/µl bis 40 µg/µl, bevorzugt 0,05 µg/µl bis 20 µg/µl, besonders bevorzugt 0,1 µg/µl bis 10 µg/µl, bezogen auf die Zusammensetzung, bezogen auf die Zusammensetzung;
(g) gegebenenfalls mindestens ein anorganisches Salz ("Komponente (g)"), insbesondere Alkali- und/oder Erdalkali-Salz, vorzugsweise Alkali- und/oder Erdalkali-Chlorid, besonders bevorzugt Natriumchlorid, insbesondere in einer Menge im Bereich von 0,01 bis 15 Gew.-%, insbesondere 0,05 bis 10 Gew.-%, vorzugsweise 0,1 bis 10 Gew.-%, bevorzugt 0,5 bis 5 Gew.-%, besonders bevorzugt 1 bis 3 Gew.-%, bezogen auf die Zusammensetzung; und
(h) gegebenenfalls mindestens ein Detergens und/oder Tensid ("Komponente (h)"), insbesondere in einer Menge im Bereich von 0,001 bis 5 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,12 bis 1 Gew.-%, bezogen auf die Zusammensetzung.

Wiederum weiterer Gegenstand der vorliegenden Erfindung gemäß einer weiteren besonders bevorzugten Ausführungsform ist zudem eine Zusammensetzung zur Verwendung bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en), vorzugsweise eine Zusammensetzung, wie sie zuvor beschrieben wurde, wobei die Zusammensetzung enthält:
(a) mindestens eine, vorzugsweise lokusspezifische, Hybridisierungssonde ("Komponente (a)"), insbesondere in einer Konzentration im Bereich von 0,1 ng/µl bis 50 ng/µl, insbesondere 0,5 ng/µl bis 50 ng/µl, vorzugsweise 0,7 ng/µl bis 8 ng/µl, bevorzugt 1 ng/µl bis 5 ng/µl, bezogen auf die Zusammensetzung;
(b) mindestens ein polares protisches oder polares aprotisches Lösemittel ("Komponente (b)"), vorzugsweise mit cyclischer Molekülstruktur, besonders bevorzugt γ-Butyrolacton, 2-Pyrrolidon (γ-Butyrolactam) und/oder Ethylencarbonat, besonders bevorzugt Ethylencarbonat, insbesondere in einer Menge im Bereich von 0,5 bis 40 Vol.-%, insbesondere 1 bis 35 Vol.-%, vorzugsweise 2 bis 30 Vol.-%, bevorzugt 5 bis 20 Vol.-%, besonders bevorzugt 7 bis 15 Vol.-%, bezogen auf die Zusammensetzung;
(c) Formamid und/oder dessen Salze ("Komponente (c)"), insbesondere in einer Menge im Bereich von 10 bis 60 Vol.-%, insbesondere 15 bis 50 Vol.-%, vorzugsweise 17 bis 40 Vol.-%, bevorzugt 20 bis 30 Vol.-%, bezogen auf die Zusammensetzung;
(d) gegebenenfalls Dextran und/oder dessen Derivate oder Salze ("Komponente (d)"), insbesondere in einer Menge im Bereich von 0,1 bis 50 Gew.-%, insbesondere 1 bis 40 Gew.-%, vorzugsweise 5 bis 30 Gew.-%, bevorzugt 10 bis 20 Gew.-%, besonders bevorzugt 13 bis 18 Gew.-%, bezogen auf die Zusammensetzung;
(e) gegebenenfalls mindestens ein chemisches Puffersystem, insbesondere in Form von Puffersalz(en), vorzugsweise Citrat und/oder Natriumchlorid, ("Komponente (e)"), insbesondere in einer Menge im Bereich von 0,001 bis 5 Gew.-%, insbesondere 0,01 bis 4 Gew.-%, vorzugsweise 0,1 bis 3 Gew.-%, bevorzugt 0,5 bis 2 Gew.-%, besonders bevorzugt 1 bis 1,5 Gew.-%, bezogen auf die Zusammensetzung und berechnet als Summe aller Bestandteile des chemischen Puffersystems;
(f) gegebenenfalls mindestens ein Blockier- und/oder Stabilisierungsmittel ("Komponente (f)"), insbesondere in einer Konzentration im Bereich von 0,001 µg/µl bis 100 µg/µl, insbesondere 0,005 µg/µl bis 80 µg/µl, vorzugsweise 0,01 µg/µl bis 40 µg/µl, bevorzugt 0,05 µg/µl bis 20 µg/µl, besonders bevorzugt 0,1 µg/µl bis 10 µg/µl, bezogen auf die Zusammensetzung, bezogen auf die Zusammensetzung;
(g) gegebenenfalls mindestens ein anorganisches Salz ("Komponente (g)"), insbesondere Alkali- und/oder Erdalkali-Salz, vorzugsweise Alkali- und/oder Erdalkali-Chlorid, besonders bevorzugt Natriumchlorid, insbesondere in einer Menge im Bereich von 0,01 bis 15 Gew.-%, insbesondere 0,05 bis 10 Gew.-%, vorzugsweise 0,1 bis 10 Gew.-%, bevorzugt 0,5 bis 5 Gew.-%, besonders bevorzugt 1 bis 3 Gew.-%, bezogen auf die Zusammensetzung; und
(h) gegebenenfalls mindestens einen Polyalkylenglycolether ("Komponente (h)"), insbesondere in einer Menge im Bereich von 0,001 bis 5 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,12 bis 1 Gew.-%, bezogen auf die Zusammensetzung.

Wie aus den vorherigen Ausführungen hervorgeht, lassen sich auf Basis der erfindungsgemäßen zielgerichteten Kombination erstmals auch geringe Mengen an Hybridisierungssonden bzw. Hybridisierungssonden in geringer Konzentration in Zusammensetzungen zur Verwendung bei der In-Situ-Hybridisierung stabilisieren. Die erfindungsgemäßen Zusammensetzungen sind sowohl für den Einsatz in automatisierten In-Situ-Hybridisierungsverfahren als auch in In-Situ-Hybridisierungsverfahren mit kurzen bzw. flexiblen Hybridisierungsdauern, wie z.B. Fast-ISH, geeignet und führen zu gut auswertbaren Signalmustern mit starken Signalen. Zusammensetzungen dieser Art sind aus dem Stand der Technik bislang nicht bekannt.

Die erfindungsgemäßen Zusammensetzungen können insbesondere für In-Situ-Hybridisierungen im Zusammenhang mit der Diagnose und/oder Prognose von Erkrankungen, insbesondere Malignomen, vorzugsweise Karzinomen, Sarkomen und/oder Leukämien, eingesetzt werden.

Die in diesem Zusammenhang zu untersuchenden Gene sind vorzugsweise ausgewählt aus der Gruppe von ALK, ROS1, RET, NRG1, NTRK1, CARS, EML4, FGFR2, FGFR3, KIF5B, TGF, BCR, ABL, ALK, BCL2, BCL6, BIRC3, CCND1, EGR1, ETV6, FGFR1, FGFR3, IGH, KMT2A, MYC, PML, RARA, RUNX1, RUNX1T1, EWSR1, CHOP, FUS, COL1A1, DDIT3, JAZF1, NR4A3, FOXO1, FUS, PAX3, PAX7, PDGFB, SS18, TFE3, USP6, WT1, HER2/ERBB2, FGFR1, ALK, CCND1, CDK4, CD274, PDCD1LG2, EGR1, EGFR, ESR1, ETV1, FGF3,4,19, FGFR2, FGFR3, FHIT (RCC), KRAS, MDM2, MDM4, MET, MYB, MYC, MYCN, PIK3CA, PTEN, SMARCB1, SOX2, TERT, TOP2A, TP53, TYMS und/oder VHL.

Besonders gute Ergebnisse werden im Rahmen der vorliegenden Erfindung erzielt, wenn die erfindungsgemäßen Zusammensetzungen zur Detektion von Chromosomenaberrationen auf Basis von Inversionen und/oder Translokationen eingesetzt werden:
In diesem Zusammenhang können die erfindungsgemäßen Zusammensetzungen insbesondere zum Nachweis unterschiedlicher Translokationen und/oder Inversionen, insbesondere in Lungentumoren eingesetzt werden, wobei insbesondere die Gene ALK, ROS1, RET, NRG1, NTRK1, CARS, EML4, FGFR2, FGFR3, KIF5B und/oder TGF betroffen sind.

Weiterhin kann es vorgesehen sein, die erfindungsgemäße Zusammensetzung zum Nachweis unterschiedlicher Translokationen und/oder Inversionen, insbesondere in Lymphomen und Leukämien einzusetzen, wobei insbesondere die Gene BCR, ABL, ALK, BCL2, BCL6, BIRC3, CCND1, EGR1, ETV6, FGFR1, FGFR3, IGH, KMT2A, MYC, PML, RARA, RUNX1 und /oder RUNX1T1 betroffen sind.

Gemäß einer weiteren bevorzugten Ausführungsform der vorliegenden Erfindung kann die erfindungsgemäße Zusammensetzung zum Nachweis unterschiedlicher Translokationen und/oder Inversionen, insbesondere in Sarkomen, eingesetzt werden, wobei insbesondere die Gene EWSR1, CHOP, FUS, COL1A1, DDIT3, JAZF1, NR4A3, FOXO1, FUS, PAX3, PAX7, PDGFB, SS18, TFE3, USP6 und/oder WT1 betroffen sind.

Auch kann es erfindungsgemäß vorgesehen sein, die Zusammensetzung zum Nachweis von Inversionen und/oder Translokationen einzusetzen, wobei insbesondere die Gene ALK und ROS1 betroffen sind.

Im Rahmen einer bevorzugten Ausführungsform der vorliegenden Erfindung kann die erfindungsgemäße Zusammensetzung auch zum Nachweis unterschiedlicher Translokationen und/oder Inversionen, insbesondere in Lungentumoren, wobei insbesondere die Gene ALK, ROS1, RET, NRG1, NTRK1, CARS, EML4, FGFR2, FGFR3, KIF5B und/oder TGF betroffen sind, eingesetzt werden.

Weiterhin kann es vorgesehen sein, dass die erfindungsgemäße Zusammensetzung zum Nachweis unterschiedlicher Translokationen und/oder Inversionen, insbesondere in Lymphomen und Leukämien, wobei insbesondere die Gene BCR, ABL, ALK, BCL2, BCL6, BIRC3, CCND1, EGR1, ETV6, FGFR1, FGFR3, IGH, KMT2A, MYC, PML, RARA, RUNX1 und /oder RUNX1T1 betroffen sind, eingesetzt wird.

Gemäß einer weiteren bevorzugten Ausführungsform der vorliegenden Erfindung wird die erfindungsgemäße Zusammensetzung zum Nachweis unterschiedlicher Translokationen und/oder Inversionen, insbesondere in Sarkomen, eingesetzt, wobei insbesondere die Gene EWSR1, CHOP, FUS, COL1A1, DDIT3, JAZF1, NR4A3, FOXO1, FUS, PAX3, PAX7, PDGFB, SS18, TFE3, USP6 und/oder WT1 betroffen sind.

Auch kann es erfindungsgemäß vorgesehen sein, dass die erfindungsgemäße Zusammensetzung zum Nachweis von Inversionen und/oder Translokationen eingesetzt wird, wobei insbesondere die Gene ALK und ROS1 betroffen sind.

Weiterer Gegenstand der vorliegenden Erfindung ist zudem - gemäß einem **zweiten** Aspekt der vorliegenden Erfindung - die Verwendung einer Zusammensetzung, wie sie zuvor beschrieben wurde, bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en).

Für weitergehende Einzelheiten zu der erfindungsgemäßen Verwendung kann auf die vorangehenden Ausführungen zu dem ersten Erfindungsaspekt, betreffend die erfindungsgemäße Zusammensetzung, verwiesen werden, welche in Bezug auf die erfindungsgemäße Verwendung entsprechend gelten.

Weiterer Gegenstand der vorliegenden Erfindung - gemäß einem **dritten** Aspekt der vorliegenden Erfindung - ist ein Verfahren zur Detektion von Nukleinsäuren, bevorzugt RNA und/oder DNA, und/oder von Chromosomenaberrationen in einer biologischen Probe, vorzugsweise in einer oder mehreren Zelle(n) und/oder in einem oder mehreren Zellkern(en) mittels Hybridisierung, insbesondere In-Situ-Hybridisierung, vorzugsweise automatisierter In-Situ-Hybridisierung, unter Verwendung einer Zusammensetzung, wie sie zuvor beschrieben wurde.

Im Rahmen der vorliegenden Erfindung ist überraschend gefunden worden, dass der Einsatz der erfindungsgemäßen Zusammensetzungen in dem erfindungsgemäßen insbesondere automatisierten Hybridisierungs-Verfahren, insbesondere In-Situ-Hybridisierungs-Verfahren, zu guten Signalmustern mit starken und gut auswertbaren Signalen führt. Darüber hinaus kann das erfindungsgemäße Verfahren zur Detektion von Nukleinsäuren unter Einsatz der Zusammensetzungen gemäß dem ersten Erfindungsaspekt überraschend auch mit kurzen Hybridisierungszeiten durchgeführt werden, ohne dass es zu einem Rückgang der Signalintensität bzw. -stärke kommt.

Insbesondere ist Gegenstand der vorliegenden Erfindung gemäß diesem Erfindungsaspekt ein Verfahren zur Detektion von Nukleinsäuren, bevorzugt RNA und/oder DNA, und/oder von Chromosomenaberrationen in einer biologischen Probe, vorzugsweise in einer oder mehreren Zelle(n) und/oder in einem oder mehreren Zellkern(en) mittels Hybridisierung, insbesondere In-Situ-Hybridisierung, vorzugsweise automatisierter In-Situ-Hybridisierung, wobei das Verfahren die folgenden Verfahrensschritte umfasst:
(a) Bereitstellen einer biologischen Probe, insbesondere auf Basis von einer oder mehreren Zelle(n) und/oder einem oder mehreren Zellkern(en), vorzugsweise in Form von Gewebe, für die In-Situ-Hybridisierung;
(b) Bereitstellen einer Zusammensetzung zur Verwendung bei der Hybridisierung, wobei die Zusammensetzung mindestens eine, vorzugsweise lokusspezifische, Hybridisierungssonde ("Komponente (a)"), mindestens ein polares protisches oder polares aprotisches Lösemittel, vorzugsweise mit cyclischer Molekülstruktur, ("Komponente (b)"), und mindestens ein Carbonsäureamid und/oder dessen Salze, insbesondere Formamid und/oder dessen Salze, in einer Menge von mehr als 10 Vol.-%, bezogen auf die Zusammensetzung ("Komponente (c)"), enthält, insbesondere einer Zusammensetzung, wie sie zuvor beschrieben wurde;
(c) Inkontaktbringen der biologischen Probe aus Verfahrensschritt (a) mit der Zusammensetzung aus Verfahrensschritt (b);
(d) Denaturierung der biologischen Probe aus Verfahrensschritt (a) und der Zusammensetzung aus Verfahrensschritt (b), wobei die biologische Probe und die Zusammensetzung getrennt voneinander, insbesondere vor Durchführung von Verfahrensschritt (c), oder gemeinsam, insbesondere nach Durchführung von Verfahrensschritt (c), denaturiert werden;
(e) nachfolgende Hybridisierung der in der Zusammensetzung enthaltenen mindestens einen, vorzugsweise lokusspezifischen, Hybridisierungssonde und den in den biologischen Proben enthaltenen Nukleinsäuren;
(f) nachfolgende Detektion der hybridisierten, vorzugsweise lokusspezifischen, Hybridisierungssonden und/oder der zu detektierenden Nukleinsäuren in der biologischen Probe.

Gemäß einer besonders bevorzugten Ausführungsform des erfindungsgemäßen Verfahrens kann es vorgesehen sein, dass dieses auf bzw. in einem Automaten und/oder automatisch durchgeführt wird. Auf dieser Basis kann die Effizienz bzw. der Probendurchsatz von Hybridisierungsverfahren, insbesondere In-Situ-Hybridisierungen, signifikant gesteigert werden.

Was Verfahrensschritt (a) bzw. die Bereitstellung einer biologischen Probe für die Durchführung des erfindungsgemäßen Verfahrens, insbesondere der In-Situ-Hybridisierung, im Speziellen anbelangt, so erfolgt dieser bzw. diese auf dem Fachmann an sich bekannte Art und Weise, d.h. die biologischen Proben werden auf an sich bekannte Art und Weise auf die In-Situ-Hybridisierung vorbereitet. Insbesondere umfasst Verfahrensschritt (a) die Fixierung und Einbettung der Proben auf Basis von Zellen bzw. Geweben, die Aufbringung der Proben auf geeignete Träger, die Probenvorbehandlung und Trocknung der Proben. Dem Fachmann ist die Bereitstellung bzw. Vorbereitung von Proben auf In-Situ-Hybridisierungs-verfahren grundsätzlich bekannt, so dass es keiner weiteren Ausführungen an dieser Stelle bedarf.

Im Zusammenhang mit Verfahrensschritt (b) bzw. der Bereitstellung der Zusammensetzung wird zur Vermeidung unnötiger Wiederholungen auf die obigen Erfindungsaspekte, insbesondere die Ausführungen im Zusammenhang mit der erfindungsgemäßen Zusammensetzung, verwiesen.

Was darüber hinaus Verfahrensschritt (c) anbelangt, hat es sich als vorteilhaft erwiesen, wenn die Zusammensetzung in einer Menge im Bereich von 0,1 bis 5.000 µl, insbesondere 1 bis 2.500 µl, vorzugsweise 2 bis 1.500 µl, bevorzugt 5 bis 1.000 µl, besonders bevorzugt 10 bis 500 µl, ganz besonders bevorzugt 20 bis 250 µl, noch mehr bevorzugt 40 bis 150 µl, eingesetzt wird. Die vorgenannten Mengen der Zusammensetzung führen sowohl bei automatisierten bzw. automatischen In-Situ-Hybridisierungen als auch bei In-Situ-Hybridisierungen mit kurzen Hybridisierungsdauern zu qualitativ hochwertigen und gut auswertbaren Signalmustern. Die Zusammensetzung wird insbesondere in einer Menge eingesetzt, die ausreicht, um den untersuchten Probenbereich zu bedecken.

Verfahrensschritt (d) zur Denaturierung der Nukleinsäuren in der biologischen Probe und in der Zusammensetzung erfolgt auf dem Fachmann grundsätzlich bekannte Art und Weise. Im Rahmen der erfindungsgemäßen Verfahrens können die biologische Probe einerseits und die Zusammensetzung andererseits getrennt voneinander vor dem Inkontaktbringen gemäß Verfahrensschritt (e) der Denaturierung unterzogen werden. Gleichermaßen kann es vorgesehen sein, die biologische Probe und die Zusammensetzung gemeinsam nach Durchführung von Verfahrensschritt (c) einer Co-Denaturierung zu unterziehen. Die Einstellung der Denaturierungsbedingungen, insbesondere der dazu erforderlichen Temperatur, ist dem Fachmann an sich bekannt und bedarf keiner weiteren Ausführungen. Insbesondere kann die Temperatur in Verfahrensschritt (d) im Bereich von 60 bis 90 °C, vorzugsweise im Bereich von 70 bis 85 °C, liegen.

Auch Verfahrensschritt (e), welcher der Hybridisierung der in den Hybridisierungszusammensetzungen enthaltenen, vorzugsweise lokusspezifischen, Hybridisierungssonden einerseits sowie der in den biologischen Proben enthaltenen zu detektierenden DNA- bzw. Chromosomenbereichen andererseits dient, erfolgt auf dem Fachmann grundsätzlich bekannte Art und Weise. Insbesondere was die Einstellung einer geeigneten Hybridisierungstemperatur anbelangt, sind keinerlei Ausführungen an dieser Stelle notwendig.

Im Zusammenhang mit Verfahrensschritt (e) hat sich im Rahmen der vorliegenden Erfindung zudem überraschend gezeigt, dass die Hybridisierung, d.h. die Anlagerung der, vorzugsweise lokusspezifischen, Hybridisierungssonden an den zu detektierenden DNA- bzw. Chromosomenabschnitt, noch weiterführend verbessert werden kann, wenn Verfahrensschritt (e) unter Bewegung, insbesondere wellenförmiger und/oder kontinuierlicher Bewegung, durchgeführt wird.

Im Rahmen der vorliegenden Erfindung hat sich zudem überraschend gezeigt, dass das erfindungsgemäße Verfahren unter Einsatz der erfindungsgemäßen Zusammensetzungen sowohl mit kurzen Hybridisierungsdauern ("Schnell-ISH"), z. B. von 10 bis 240 min, als auch mit den üblicherweise eingesetzten langen Hybridisierungsdauern, z. B. 4 bis 100 h, zu hervorragenden Ergebnissen führt.

So kann es im Rahmen der vorliegenden Erfindung vorgesehen sein, dass im Rahmen des erfindungsgemäßen Verfahrens die Hybridisierung bzw. Verfahrensschritt (e) über eine Dauer im Bereich von 10 min bis 240 min, insbesondere im Bereich von 30 min bis 180 min, vorzugsweise im Bereich von 60 min bis 150 min, bevorzugt im Bereich von 90 min bis 130 min, durchgeführt wird. Darüber hinaus kann es auch vorgesehen sein, dass die Hybridisierung bzw. Verfahrensschritt (e) über eine Dauer im Bereich von 1 h bis 100 h, insbesondere 2 h bis 80 h, vorzugsweise 3 h bis 50 h, bevorzugt 4 h bis 30 h, besonders bevorzugt 5 h bis 25 h, noch mehr bevorzugt 8 h bis 20 h, durchgeführt wird.

Die Detektion der gebundenen bzw. hybridisierten, vorzugsweise lokusspezifischen, Hybridisierungssonden in der biologischen Probe erfolgt ebenfalls mit dem Fachmann an sich bekannten Methoden in Abhängigkeit von der Markierung der Hybridisierungssonden. Diesbezüglich bedarf es keinerlei weiterer Ausführungen. Im Rahmen des erfindungsgemäßen Verfahrens werden bevorzugt fluoreszenzmarkierte Hybridisierungssonden (Fluoreszente ISH (FISH)), welche fluoreszenzmikroskopisch detektiert werden, oder antigenmarkierte Sonden, insbesondere Hapten-markierte Sonden, eingesetzt, welche mit Hilfe von Antikörpern durch Farbreaktionen sichtbar gemacht und lichtmikroskopisch detektiert werden ((Hellfeld ISH (BrISH), Chromogene ISH (CISH), Silber ISH (SISH)), eingesetzt. Wie genau die Detektion der eingesetzten markierten Hybridisierungssonden im Rahmen des erfindungsgemäßen Verfahrens zu erfolgen hat, versteht sich für den Fachmann von selbst.

Für weitergehende Einzelheiten zu dem erfindungsgemäßen Verfahren kann auf die vorangehenden Ausführungen zu den obigen Erfindungsaspekten verwiesen werden, welche in Bezug auf das erfindungsgemäße Verfahren entsprechend gelten.

Schließlich ist Gegenstand der vorliegenden Erfindung - gemäß einem **vierten** Aspekt der vorliegenden Erfindung - ein Kit bzw. Kit-of-parts bzw. System zur Detektion von Nukleinsäuren, bevorzugt RNA und/oder DNA, und/oder von Chromosomenaberrationen in einer biologischen Probe, vorzugsweise in einer oder mehreren Zelle(n) und/oder in einem oder mehreren Zellkern(en) mittels Hybridisierung, insbesondere In-Situ-Hybridisierung, vorzugsweise automatisierter In-Situ-Hybridisierung, wobei das Kit eine Zusammensetzung, wie sie zuvor beschrieben wurde, enthält und/oder wobei das Kit zur Durchführung des zuvor geschilderten Verfahrens bestimmt ist und/oder eingesetzt wird und/oder geeignet ist.

Gemäß einer bevorzugten Ausführungsform kann es erfindungsgemäß vorgesehen sein, dass das erfindungsgemäße Kit die Komponenten der Zusammensetzung, wie sie zuvor beschrieben wurde, in einem gemeinsamen Aufbewahrungsgefäß oder Anwendungsvorrichtung oder in räumlich getrennten, voneinander verschiedenen Aufbewahrungsgefäßen und/oder Anwendungsvorrichtungen enthält.

Für weitergehende Einzelheiten zu dem erfindungsgemäßen Kit kann auf die vorangehenden Ausführungen zu den obigen Erfindungsaspekten verwiesen werden, welche in Bezug auf das erfindungsgemäße Kit entsprechend gelten.

### Kurze Beschreibung der Figuren

Weitere Merkmale, Vorteile und Besonderheiten der vorliegenden Erfindung ergeben sich aus der nachfolgenden Beschreibung von bevorzugten Ausführungsformen anhand der Figuren 1 bis 4.

Es zeigen:
- Fig.1: eine schematische Darstellung von In-Situ-Hybridisierungs-Ergebnissen betreffend die Signalmuster, insbesondere die Signalstärke.
- Fig. 2: eine schematische Darstellung der Signalmuster und insbesondere Signalstärken bei Verwendung einer FISH-Sonde "Auto ALK Break Apart Probe" im Rahmen von In-Situ-Hybridisierungen unter Einsatz von Färbeautomaten.
- Fig. 3: eine schematische Darstellung der Signalmuster und insbesondere Signalstärken bei Verwendung einer flexiblen FISH-Sonde "Flexible ROS1 Break Apart Probe" im Rahmen von In-Situ-Hybridisierungen.
- Fig. 4: eine schematische Darstellung der Signalmuster und insbesondere Signalstärken bei Verwendung einer flexiblen FISH-Sonde "Flexible HER2/CEN17 Probe" im Rahmen von In-Situ-Hybridisierungen.

### Ausführlichere Beschreibung der Figuren

Aufgrund der besseren Darstellbarkeit in Schwarz/Weiß, stellen die hier gezeigten Figuren keine Fluoreszenzabbildungen der unten beschriebenen Versuchsergebnisse dar, sondern Schemata, die unter anderem anhand der jeweils beobachteten Ergebnisse erstellt wurden.

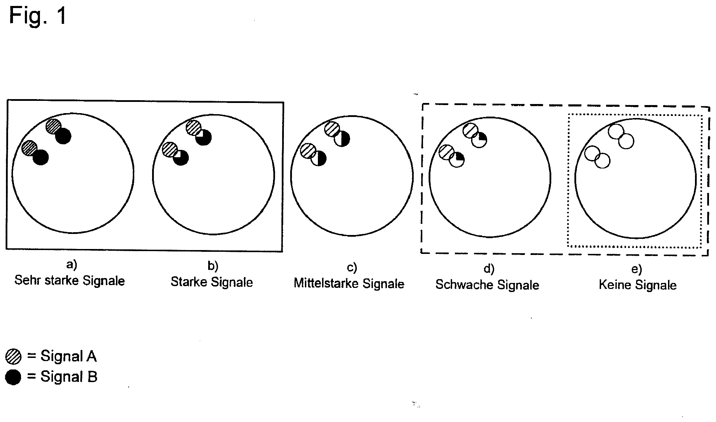

Fig. 1. zeigt eine schematische Darstellung von In-Situ-Hybridisierungs-Ergebnissen (exemplarisch sowohl für FISH als auch BrISH und CISH), welche Rückschlüsse auf das erhaltene Signalmuster und insbesondere die Signalstärken erlaubt. Als lokusspezifische Hybridisierungssonden können insbesondere Sonden, welche spezifisch für genomische Bereiche der menschlichen Gene HER2, ALK oder ROS1 sind, eingesetzt werden.

Grundsätzlich wird im Rahmen der vorliegenden Erfindung von diploiden Zellen ausgegangen, d.h. je detektierter genomischer Region werden zwei Signale bzw. ein Doppelsignal in Zellen ohne Aberrationen erhalten. Die erhaltenen Signalstärken werden in die folgenden fünf Abstufungen unterteilt: a) sehr starke Signale, b) starke Signale, c) mittelstarke Signale, d) schwache Signale, e) keine Signale, vgl. Fig. 1). Nachfolgend werden Beispiele von Signalmustern, insbesondere Signalstärken, von In-Situ-Hybridisierungen mit Standard-Hybridisierungslösungen einerseits und erfindungsgemäßen Hybridisierungslösungen andererseits bzw. von In-Situ-Hybridisierungen nach dem erfindungsgemäßen Verfahren und nach Standardverfahren gezeigt.

Die erfindungsmäßigen Verfahren und Zusammensetzungen auf Basis von spezifischen Konzentrationen von Formamid und polarem protischem oder polarem aprotischem Lösemittel mit cyclischer Molekülstruktur führen zu sehr starken oder starken Signale (Fig. 1 a) und b)). Diese hervorragenden Signalmuster werden sowohl bei flexiblen In-Situ-Hybridisierungen, d.h. bei In-Situ-Hybridisierungen mit zweistündiger Hybridisierungsdauer ("Fast-ISH") oder aber mit 16 Stunden Hybridisierungsdauer, als auch bei automatisierten Verfahren zur Durchführung der In-Situ-Hybridisierung beobachtet. Dabei ist die Signalstärke als Ergebnis sowohl der Brillanz als auch des Kontrastes zum Hintergrund zu sehen (beispielsweise führt ein starker Hintergrund zu einem schwächeren Kontrast und somit zu weniger starken Signalen).

Zusammensetzungen bzw. Hybridisierungslösungen, welche ein polares protisches oder polares aprotisches Lösemittel mit cyclischer Molekülstruktur, jedoch kein Formamid enthalten, führen zu keinen Hybridisierungssignalen (Fig. 1 e)). Zusammensetzungen bzw. Hybridisierungslösungen, welche Formamid, jedoch kein polares protisches oder polares aprotisches Lösemittel mit cyclischer Molekülstruktur enthalten, führen ebenfalls zu keinen Hybridisierungssignalen oder allenfalls zu sehr schwachen bzw. kaum detektierbaren Hybridisierungssignalen (Fig. 1 e) und f)).

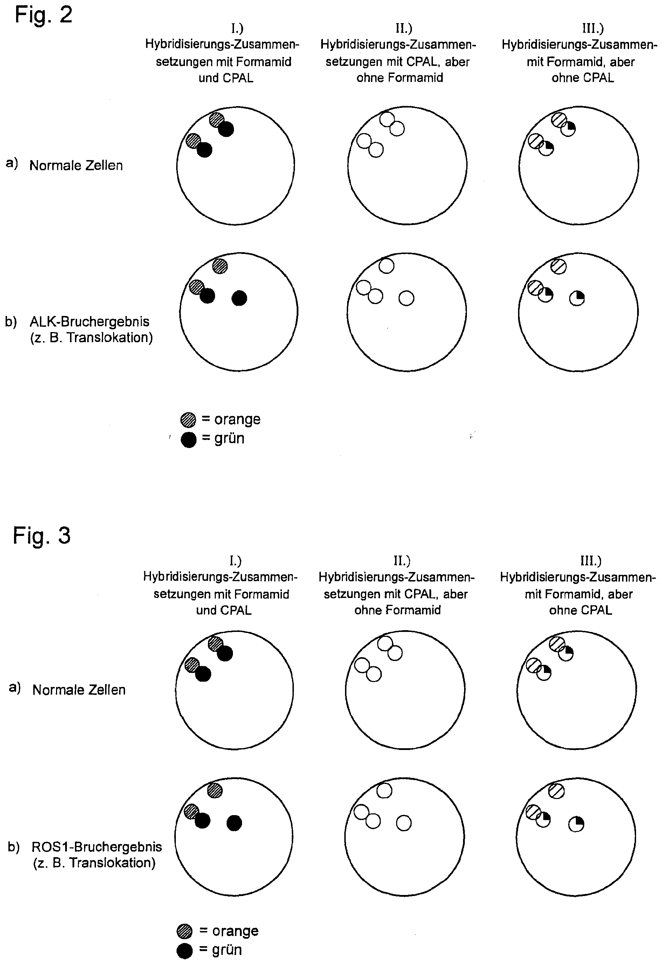

Fig. 2 zeigt eine schematische Darstellung der Signalmuster und Signalstärken von In-Situ-Hybridisierungen, welche unter Verwendung einer FISH-Sonde "Auto ALK Break Apart Probe" auf einem Färbeautomaten durchgeführt werden. Diese lokusspezifische Hybridisierungssonde besteht aus grün-markierten Polynukleotiden (Absorption bei 503 nm und Emission bei 528 nm), die in 2p23 gegen proximal zur ALK-Bruchpunktregion gelegene Sequenzen gerichtet sind, und orange-markierten Polynukleotiden (Absorption bei 547 nm und Emission bei 572 nm), die in 2p23 gegen distal zur ALK-Bruchpunktregion gelegene Sequenzen gerichtet sind. Die Hybridisierungslösung bzw. -zusammensetzung basiert auf den nachfolgend aufgeführten Komponenten: 18 Gew.-% Dextransulfat, 600 mM NaCl, 22 Vol.-% Formamid, 1-fach konzentriertem SSC-Puffer, 12,5 Gew.-% Ethylencarbonat, Blockier- und Stabilisierungs-DNA in einer Konzentration von 0,1 µg/µl und lokusspezifische Hybridisierungssonden in einer Konzentration von 2 ng/µl, jeweils bezogen auf die Hybridisierungslösung bzw. - zusammensetzung.

Die Hybridisierungssonde wird nach der Denaturierung (20 Minuten bei 75 °C) über eine Hybridisierungs- bzw. Renaturierungsdauer von 120 Minuten bei 45 °C in einem Automaten (Celerus Wave RPD System) unter ständiger Wellenbewegung der Objektträger mit Zell- und Gewebeproben hybridisiert.

Bei Verwendung geeigneter Filtersätze erscheinen die Hybridisierungssignale für das nicht-rearrangierte ALK-Gen als grün-orange Fluoreszenz-Fusionssignale. In der Interphase einer normalen Zelle (ohne ALK-Aberration) erscheinen zwei grün-orange Doppel- bzw. Fusionssignale bei Verwendung eines geeigneten grün-orange-Dual-Bandpass-Filtersets (Fig. 2 la). Ein von einer ALK-Translokation betroffener 2p23-Lokus ist durch ein separates grünes Signal und ein separates oranges Signal gekennzeichnet (Fig. 2 Ib).

Die erhaltenen Signalstärken und somit die Auswertbarkeit der Ergebnisse hängen entscheidend von den spezifischen Konzentrationen der polaren protischen oder polaren aprotischen Lösemittel mit cyclischer Molekülstruktur (CPAL) und des Formamids in den zugrunde liegenden Hybridisierungslösungen der Hybridisierungssonden ab. Bei niedrigen Konzentrationen der polaren protischen oder polaren aprotischen Lösemittel mit cyclischer Molekülstruktur (CPAL), z.B. wie hier Ethylencarbonat, zwischen 5 Gew.-% und 13 Gew.-% und Formamid-Konzentrationen größer als 10 Vol.-% erscheinen sehr starke Fluoreszenzsignale bei sehr geringem Hintergrund (Fig. 2 I). Vergleichszusammensetzungen, welche kein Formamid enthalten, führen zu keinen auswertbaren Ergebnissen, d.h. es können keine genspezifischen Signale generiert werden (Fig. 2 II). Vergleichszusammensetzungen, welche kein polares protisches oder polares aprotisches Lösemittel mit cyclischer Molekülstruktur, z.B. Ethylencarbonat, enthalten, führen zu sehr schwachen und somit schlecht auswertbaren Signalen (Fig. 2 III).

Fig. 3 zeigt eine schematische Darstellung der Signalmuster und Signalstärken von In-Situ-Hybridisierungen, welche unter Verwendung einer FISH-Sonde "Flexible ROS1 Break Apart Probe" durchgeführt werden. Diese lokusspezifische Hybridisierungssonde besteht aus grün-markierten Polynukleotiden (Absorption bei 503 nm und Emission bei 528 nm), die in 6q22 gegen proximal zur ROS1-Bruchpunktregion gelegene Sequenzen gerichtet sind, und orange-markierten Polynukleotiden (Absorption bei 547 nm und Emission bei 572 nm), die in 6q22 gegen distal zur ROS1-Bruchpunktregion gelegenen Sequenzen gerichtet sind. Die Hybridisierungslösung bzw. -zusammensetzung basiert auf den nachfolgenden Komponenten: 15 Gew.-% Dextransulfat, 500 mM NaCl, 27 Vol.-% Formamid, 1-fach konzentriertem SSC-Puffer, 9,75 Gew.-% Ethylencarbonat, Blockier- und Stabilisierungs-DNA in einer Konzentration von 2 µg/µl und lokusspezifischen Hybridisierungssonden in einer Konzentration von 5 ng/µl, jeweils bezogen auf die Hybridisierungslösung bzw. -zusammensetzung. Die Hybridisierungssonden werden nach Denaturierung (10 Minuten bei 75 °C) über eine Hybridisierungs- bzw. Renaturierungsdauer von 10 Minuten entweder 2 Stunden lang bei 37 °C oder aber 16 Stunden lang bei 37 °C mit Zell- und Gewebeproben hybridisiert.

Bei Verwendung geeigneter Filtersätze erscheinen die Hybridisierungssignale für das nicht-rearrangierte ROS1-Gen als grün-orange Fluoreszenz-Signale. In der Interphase einer normalen Zelle (ohne ROS1-Aberration) erscheinen zwei grün-orange Fusions- bzw. Doppelsignale bei Verwendung eines geeigneten grün-orange-Dual-Bandpass-Filtersets (Fig. 3 la). Ein von einer ROS1-Translokation betroffener 6q22-Lokus ist durch ein separates grünes Signal und ein separates oranges Signal gekennzeichnet (Fig. 3 Ib).

Die erhaltenen Signalstärken und somit die Auswertbarkeit der Ergebnisse hängen entscheidend von den spezifischen Konzentrationen der polaren protischen oder polaren aprotischen Lösemittel mit cyclischer Molekülstruktur (CPAL) und des Formamids in den zugrundeliegenden Hybridisierungslösungen der lokusspezifischen Hybridisierungssonde ab. Bei niedrigen Konzentrationen der polaren protischen oder polaren aprotischen Lösemittel mit cyclischer Molekülstruktur (CPAL), z.B. wie hier Ethylencarbonat, im Bereich von 5 Gew.-% und 13 Gew.-% sowie Formamid-Konzentrationen von mehr als 10 Gew.-% erscheinen sehr starke Fluoreszenzsignale bei sehr geringem Hintergrund sowohl nach einer Hybridisierungszeit von zwei Stunden als auch von 16 Stunden (Fig. 3 I). Hinsichtlich der Signalstärke werden mit den erfindungsgemäßen Hybridisierungslösungen sowohl bei einer kurzen als auch bei einer langen Hybridisierungsdauer hervorragende Ergebnisse erzielt.

Vergleichszusammensetzungen, welche ein polares protisches oder polares aprotisches Lösemittel mit cyclischer Molekülstruktur, jedoch kein Formamid enthalten, führen sowohl nach zwei als auch nach 16 Stunden Hybridisierungsdauer zu keinen auswertbaren Ergebnissen, d.h. es können keine genspezifischen Signale generiert werden (Fig. 3 II). Vergleichszusammensetzungen, welche Formamid, jedoch kein polares protisches oder polares aprotisches Lösemittel mit cyclischer Molekülstruktur (CPAL) enthalten, führen zu sehr schwachen Signalen (Fig. 3 III).

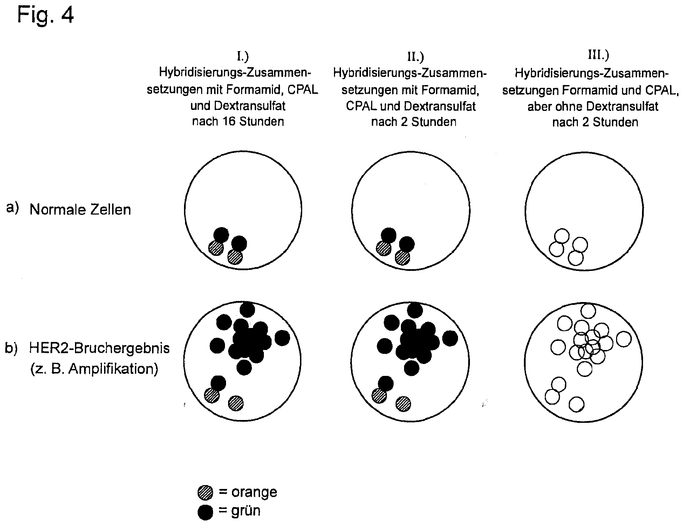

Fig. 4 zeigt eine schematische Darstellung der Signalmuster und Signalstärken von In-Situ-Hybridisierungen, welche unter Verwendung einer flexiblen FISH-Sonde "Flexible HER2/CEN 17 Probe" durchgeführt wurden. Diese Sonde besteht aus grün-markierten Polynukleotiden (Absorption bei 503 nm und Emission bei 528 nm), die gegen die Region 17q11.2-q12 des HER2-Gens gerichtet sind, und orange-markierten Polynukleotiden (Absorption bei 547 nm und Emission bei 572 nm), die gegen die Alpha-Satelliten-Zentromer-Region von Chromosom 17 (D17Z1) gerichtet sind. Die Hybridisierungslösung enthält die nachfolgend aufgeführten Komponenten: 15 Gew.-% Dextransulfat, 500 mM NaCl, 27 Vol.-% Formamid, 1-fach konzentrierten SSC-Puffer, 10,5 Gew.-% Ethylencarbonat, Blockier- und Stabilisierungs-DNA in einer Konzentration von 2 µg/µl und lokusspezifische Hybridisierungssonden in einer Konzentration von 5 ng/µl Sonden-DNA, jeweils bezogen auf die Zusammensetzung. Die Hybridisierungssonden werden nach Denaturierung (10 Minuten bei 75 °C) entweder zwei Stunden lang bei 37 °C oder aber 16 Stunden lang bei 37 °C mit Zell- und Gewebeproben hybridisiert.

Bei Verwendung geeigneter Filtersätze erscheinen in dem erzeugten Signalmuster die Hybridisierungssignale für das nicht-rearrangierte HER2-Gen als zwei grüne Fluoreszenz-Signale und die Hybridisierungssignale für die nicht-rearrangierte Zentromer-Region von Chromosom 17 als zwei orange Fluoreszenz-Signale. In der Interphase einer normalen Zelle ohne HER2-Aberration und ohne Aberrationen von Chromosom 17 erscheinen zwei grüne und zwei orange Signale bei Verwendung eines geeigneten grün-orange-Dual-Bandpass-Filtersets (Fig. 4 Ia und IIa). Ein von einer HER2-Amplifikation betroffener 17q11.2-q12-Lokus ist durch zusätzliche weitere grüne Signale gekennzeichnet (Fig. 4 Ib und IIb).

Die erhaltenen Signalstärken und somit die Auswertbarkeit der Ergebnisse hängen entscheidend von dem Vorhandensein von Dextransulfat in den zugrundeliegenden Hybridisierungslösungen bzw. -zusammensetzungen ab. Mit Hybridisierungslösungen, welche ein polares protisches bzw. polares aprotisches Lösemittel mit cyclischer Molekülstruktur, z.B. Ethylencarbonat, in einer Menge im Bereich von 5 Gew.-% bis 13 Gew.-%, Formamid in einer Menge von mindestens 10 Gew.-% und Dextransulfat in einer Menge von 15 Gew.-% enthalten, werden im Rahmen der In-Situ-Hybridisierungen gleichermaßen sowohl bei kurzen als auch bei langen Hybridisierungsdauern von zwei bzw. 16 Stunden sehr starke Fluoreszenzsignale bei sehr geringem Hintergrund erzielt (Fig. 4 I und II). Hybridisierungslösungen, welche kein Dextransulfat enthalten, führen zu keinen auswertbaren Ergebnissen, d.h. es können keine genspezifischen Signale generiert werden (Fig. 4 III).

### Ausführungsbeispiele

**A)** Bevor einzelne, insbesondere besonders bevorzugte Ausführungsformen der vorliegenden Erfindung beschrieben werden, werden zunächst die zugrundeliegenden In-Situ-Hybridisierungsverfahren als solche beschrieben.

### Beispiel A.1 ISH-Prozedur I (Flexible FISH mit Hybridisierungsdauern von zwei Stunden oder 16 Stunden)

Die zur Durchführung der "flexiblen" Schnell-FISH verwendeten Reagenzien stammten aus dem ZytoLight Flexible FISH-Tissue Implementation Kit (Z-2182-20, ZytoVision GmbH, Bremerhaven, Deutschland). Das Kit enthält die nötigen Reagenzien (Heat Pretreatment Solution Citric, Pepsin Solution, 5-fach konzentrierten Flexible FISH Wash Buffer und DAPI/DuraTect^{™}-Solution) zur Durchführung der FISH an Schnitten von mit Formalin fixierten und in Paraffin eingebetteten Geweben.

Die Durchführung der FISH erfolgte an 3 bis 5 µm dicken Schnitten von Formalin-fixierten Paraffin-eingebetteten (FFPE) Geweben aus Mammakarzinomen, Lungengewebe, Lymphknotengewebe, Nierengewebe, Prostatagewebe bzw. Plazentagewebe. Die Gewebeschnitte wurden auf beschichtete Objektträger aufgezogen und über Nacht bei 58 °C gebacken.

Zur Entfernung des Paraffins wurden die Präparate zweimal für jeweils fünf Minuten bei Raumtemperatur (RT) in 100 % Xylol inkubiert. Danach wurde eine absteigende Ethanolreihe für jeweils zwei Minuten bei Raumtemperatur durchgeführt (zweimal jeweils 96 %, 90 %, 70 % vergällter Ethanol). Nach zwei Inkubationsschritten für jeweils zwei Minuten in Reinstwasser bei Raumtemperatur schloss sich die Hitzevorbehandlung über eine Dauer von 20 Minuten bei 98 °C in Heat Pretreatment Solution Citric an, gefolgt von zwei weiteren Inkubationsschritten für jeweils zwei Minuten in Reinstwasser bei RT. Die proteolytische Vorbehandlung erfolgte durch Auftropfen von Pepsin Solution (RTU) auf die Präparate und anschließende Inkubation in einer feuchten Kammer bei 37 °C für eine Zeit von 5 bis 30 Minuten. Dem Verdau folgten zwei Inkubationen für je zwei Minuten bei RT in Reinstwasser und eine aufsteigende Ethanolreihe (70 %, 90 %, 96 %) für je eine Minute bei RT.

Nach der Lufttrocknung der Präparate wurden 10 µl einer Hybridisierungslösung, welche die fluoreszenzmarkierten Hybridisierungssonden enthielt, mittels Pipette direkt auf die Schnitte aufgetragen. Nach dem Auflegen eines geeigneten Deckgläschens wurden die Präparate mit Fixogum versiegelt und zur Co-Denaturierung für zehn Minuten bei 75 °C auf einer Heizplatte gelagert. Zur Hybridisierung wurden die Präparate in eine vorgewärmte feuchte Kammer überführt und bei 37 °C entweder für zwei Stunden oder über Nacht (ca. 16 Stunden) inkubiert.

Vor der Stringenzwaschung wurde das Fixogum entfernt und die Präparate wurden für ca. zwei Minuten bei RT in 1-fach konzentriertem Flexible Wash Buffer inkubiert. Danach wurde das Deckgläschen entfernt, und die eigentliche Waschung erfolgte durch Inkubation in 1-fach konzentriertem Flexible Wash Buffer über eine Dauer von zehn Minuten bei 72 °C und eine weitere Inkubation über drei Minuten bei RT. Es schloss sich dann eine aufsteigende Ethanolreihe (70 %, 90 %, 96 %) für jeweils eine Minute bei RT an, bevor die Präparate luftgetrocknet wurden, wobei die Trocknung unter Ausschluss von Licht erfolgte. Abschließend wurde DAPI DuraTect Solution aufgetragen und das Präparat mit einem Deckgläschen versehen.

Die Auswertung erfolgte unter Verwendung eines Fluoreszenzmikroskops (Axio Scope.A1 mit Beleuchtungseinheit HXP 120V, Carl Zeiss Microscopy GmbH) und entsprechender Filtersätze für die jeweils zugrundeliegenden Absorptions- und Emissionsbereiche.

### Beispiel A.2 ISH-Prozedur II (Automatisierte FISH auf dem Automaten Celerus Wave RPD System)

Die zur Durchführung der automatisierten FISH (auf dem Automaten Celerus Wave RPD System der Firma Celerus Diagnostics, California, USA) verwendeten Reagenzien waren im LRM Bin (Celerus Diagnostics, California, USA) enthalten. Das LRM (Linear Reagent Magazine) enthielt alle nötigen Reagenzien für die Hitzevorbehandlung, die Proteolyse sowie den Waschpuffer für die nötigen Waschschritte. Zum Eindecken der Präparate und Anfärbung der Zellkerne wurde DAPI/DuraTect^{™} (ZytoVision GmbH, Bremerhaven, Deutschland) verwendet. Die Automaten-Sonde befand sich im PAC Bin, welcher in den LRM Bin eingesetzt wurde.

Die Durchführung der automatisierten FISH erfolgte an 3 bis 5 µm dicken Schnitten mit Formalin fixierten und in Paraffin eingebetteten (FFPE) humanen Geweben aus Mammakarzinomen, Lunge, Lymphknoten, Nieren, Prostata und/oder Plazenta, die auf beschichtete Objektträger aufgezogen und über Nacht bei 58 °C gebacken wurden. Zur Entfernung des Paraffins wurden die Präparate nach dem vorgegebenen Programm des Celerus Wave^{®} RPD System behandelt. Anschließend folgte die Hitzevorbehandlung für 15 Minuten bei 95 °C, gefolgt von zwei Inkubationsschritten in Waschpuffer für sechs Minuten bei 40 °C. Nach einer Trocknung für 10 Minuten bei 45 °C schloss sich die proteolytische Vorbehandlung an. Diese erfolgte mit Pepsin durch Inkubation der Präparate über eine Dauer im Bereich von 5 bis 40 Minuten bei 60 °C. Dem Verdau folgten zwei Inkubationen in Reinstwasser von jeweils vier Minuten bei 37 °C und anschließend ein Trocknungsschritt von sieben Minuten bei 50 °C.

Nach der Trocknung erfolgte die Auftragung der fluoreszenzmarkierten lokusspezifischen Hybridisierungssonde, welche in der Hybridisierungslösung vorlag. Dazu wurden 130 µl der Hybridisierungslösung pro Objektträger auf die Präparate aufgetragen. Die Co-Denaturierung erfolgte über eine Dauer von zehn Minuten bei 80 °C und die anschließende Hybridisierung erfolgte für 120 Minuten bei 42 °C. Die Hybridisierung wurde durch permanente Bewegungen der Objektträger und somit wellenförmige Bewegungen der Hybridisierungslösung begleitet. Nach der Hybridisierung erfolgte die Waschung mit Waschpuffer für vier Minuten bei 37 °C.

Die Stringenzwaschung erfolgte über eine Dauer von 15 Minuten bei 45 °C. Eine abschließende Waschung wiederum erfolgte für vier Minuten bei 37 °C. Abschließend erfolgte eine Trocknung für fünf Minuten bei 45 °C auf dem Automaten. Nach der automatischen Durchführung dieser Schritte erfolgte die Entnahme der Präparate aus dem Automaten. Es schloss sich eine aufsteigende Ethanolreihe (70 %, 90 %, 96 %) für je eine Minute bei RT an, bevor die Präparate unter Ausschluss von Licht luftgetrocknet wurden. Zuletzt wurde eine Färbung der Zellkerne mit Hilfe des Farbstoffs 4',6-Diamidin-2-phenylindol (DAPI) durch den Auftrag von DAPI DuraTect Solution (ZytoVision GmbH, Bremerhaven) vorgenommen und das Präparat mit einem Deckgläschen versehen.

Die Auswertung erfolgte unter Verwendung eines Fluoreszenzmikroskops (Axio Scope.A1 mit Beleuchtungseinheit HXP 120V, Carl Zeiss Microscopy GmbH) und entsprechender Filtersätze für die jeweils zugrundeliegenden Absorptions- und Emissionsbereiche.

### Beispiel A.3 ISH-Prozedur III (Automatisierte FISH auf dem Automaten Pathcom Stainer)

Die zur Durchführung der automatisierten FISH (auf dem Automaten Pathcom Stainer der Firma PathCom Systems Cooperation, California, USA) verwendeten Reagenzien stammten aus dem ISH Detection Kit (PathCom Systems Corporation, Sierra Ct, Dublin, USA). Das Kit enthielt alle nötigen Reagenzien (Dewax Solutions, Retrieval Solution, Pepsin, diH₂O) zur Durchführung der automatisierten FISH. Weiterhin wurde der PathCom Systems Wash Buffer for IHC und ISH benötigt. Zum Eindecken der Präparate und Anfärbung der Zellkerne wurde DAPI/DuraTect^{™} Solution (ZytoVision GmbH, Bremerhaven, Deutschland) verwendet.

Die Durchführung der automatisierten FISH erfolgte an 3 bis 5 µm dicken Schnitten von mit Formalin fixierten und in Paraffin eingebetteten (FFPE) humanen Geweben, z.B. aus Mammakarzinomen, Lunge, Lymphknoten, Nieren, Prostata und/oder Plazenta, die auf beschichtete Objektträger aufgezogen und über Nacht bei 58 °C gebacken wurden.

Zur Entfernung des Paraffins wurden die Präparate zunächst über eine Dauer von sechs Minuten bei 65 °C mit einer ersten Dewax Solution behandelt. Danach erfolgte die Behandlung mit vier weiteren Dewax Solutions für jeweils sechs Minuten bei 62 °C und abschließend mit einer finalen Dewax Solution für sechs Minuten bei 50 °C. Es folgte die Hitzevorbehandlung mit der Retrieval Solution zunächst für 15 Minuten bei 98 °C und anschließend für acht Minuten bei 65 °C, so dass die gesamte Hitzevorbehandlung ungefähr 23 Minuten andauerte. Nach zwei Inkubationsschritten mit einem Waschpuffer ("Wash Buffer") für jeweils sechs Minuten bei 40 °C erfolgt eine Trocknung für zehn Minuten bei 45 °C. Der Trocknung schloss sich eine proteolytische Vorbehandlung an. Diese erfolgte durch Inkubation der Präparate in Pepsin über eine Dauer im Bereich von 5 bis 40 Minuten bei 37 °C. Dem Verdau folgten drei Inkubationen in Reinstwasser für je drei Minuten bei 37 °C und anschließend ein Trocknungsschritt für zehn Minuten bei 50 °C.

Nach der Trocknung erfolgte die Auftragung von 110 µl Hybridisierungslösung, welche die fluoreszenzmarkierten lokusspezifischen Hybridisierungssonden enthielt, auf die Präparate. Die Co-Denaturierung von Probe und Hybridisierungslösung erfolgte über eine Dauer von 20 Minuten bei 75 °C. Die sich an die Denaturierung anschließende Hybridisierung bzw. Renaturierung wurde über eine Dauer von 120 Minuten bei 45 °C unter kontinuierlicher Bewegung der Hybridisierungslösung durch induzierte Bewegung der Reaktionskammer im Automaten durchgeführt.

Nach der Hybridisierung erfolgte die Waschung mit Waschpuffer ("Wash Buffer") für vier Minuten bei 37 °C. Die Stringenzwaschung wurde über eine Dauer von 15 Minuten bei 45 °C durchgeführt. Eine abschließende Waschung wiederum erfolgte für vier Minuten bei 37 °C. Abschließend erfolgte eine Trocknung für fünf Minuten bei 45 °C auf dem Automaten. Nach der automatischen Durchführung dieser Schritte erfolgte die Entnahme der Präparate aus dem Automaten. Es schloss sich eine aufsteigende Ethanolreihe (70 %, 90 %, 96 %) für je eine Minute bei RT an, bevor die Präparate unter Ausschluss von Licht luftgetrocknet wurden. Abschließend erfolgte eine Färbung der Zellkerne mit dem Farbstoff 4',6-Diamidin-2-phenylindol (DAPI) durch den Auftrag von "DAPI DuraTect^{™} Solution" (ZytoVision GmbH, Bremerhaven) und das Präparat wurde mit einem Deckgläschen versehen.

Die Auswertung erfolgte unter Verwendung eines Fluoreszenzmikroskops (Axio Scope.A1 mit Beleuchtungseinheit HXP 120V, Carl Zeiss Microscopy GmbH) und entsprechender Filtersätze für die jeweils zugrunde liegenden Absorptions- und Emissionsbereiche.

**B)** Nachfolgend werden erfindungsgemäße, insbesondere besonders bevorzugte Ausführungsformen der vorliegenden Erfindung sowie vergleichende In-Situ-Hybridisierungen beschrieben:

### Beispiel B.1 FISH zum Nachweis genspezifischer HER2-Signale in einem "flexiblen" Schnell-FISH-Verfahren

Unter Einsatz der lokusspezifischen Hybridisierungssonde "Flexible HER2/CEN17 Probe" wurden genspezifische HER2-Signale mit Hilfe eines "flexiblen" Schnell-FISH-Verfahrens, welches sowohl mit kurzen Hybridisierungsdauern von zwei Stunden als auch mit einer Hybridisierung über Nacht von 16 Stunden durchgeführt werden kann, erzeugt bzw. nachgewiesen. Die Hybridisierung als solche wurde gemäß der oben beschriebenen ISH-Prozedur I (Flexible FISH mit Hybridisierungsdauern von zwei Stunden oder 16 Stunden) durchgeführt.

Die Hybridisierungssonde bestand aus grün-markierten Polynukleotiden (Absorption bei 503 nm und Emission bei 528 nm), die gegen die Region 17q11.2-q12 des HER2-Gens gerichtet waren, und orange-markierten Polynukleotiden (Absorption bei 547 nm und Emission bei 572 nm), die gegen die Alpha-Satelliten-Zentromer-Region von Chromosom 17 (D17Z1) gerichtet waren.

Hybridisierungslösung I: 15 Gew.-% Dextransulfat, 500 mM NaCl, 27 Vol.-% Formamid, 1-fach konzentrierter SSC-Puffer, 12 Gew.-% Ethylencarbonat, 2 µg/µl Blockier- und Stabilisierungs-DNA und 5 ng/µl lokusspezifische Hybridisierungssonde, jeweils bezogen auf die Zusammensetzung.

Hybridisierungslösung II: 15 Gew.-% Dextransulfat, 500 mM NaCl, 27 Vol.-% Formamid, 1-fach konzentrierter SSC-Puffer, 10,5% Gew.-% Ethylencarbonat, 2 µg/µl Blockier- und Stabilisierungs-DNA und 5 ng/µl lokusspezifische Hybridisierungssonde, jeweils bezogen auf die Zusammensetzung.

Beide Hybridisierungslösungen I und II führten gleichermaßen zu hervorragenden Ergebnissen, d.h. sehr starken Signalen. Es zeigten sich sehr starke genspezifische HER2-Signale neben ebenfalls sehr starken CEN17-spezifischen Signalen bei sehr geringem Hintergrund und gut erhaltener Struktur der Zellen und Gewebe sowohl bei kurzen Hybridisierungsdauern von zwei Stunden als auch bei langen Hybridisierungsdauern von 16 Stunden. Dies konnte sowohl bei normalen Zellen/Geweben bzw. Zellen/Geweben ohne Aberrationen an Chromosom 17 als auch bei Zellen/Geweben, insbesondere aus Mammakarzinomen, mit HER2-Amplifikationen beobachtet werden. Somit konnten die mit der Hybridisierungssonde zu identifizierenden HER2-Amplifikationen, welche in dem Signalmuster anhand des Auftretens mehrerer HER2-spezifischer Signale oder HER2-Signal-Cluster identifiziert wurden (vgl. Fig. 4), eindeutig detektiert werden.

### Beispiel B.2 Einfluss der Formamid-Konzentration auf flexible Schnell-FISH-Verfahren

Unter Einsatz der lokusspezifischen Hybridisierungssonde "Flexible HER2/CEN17 Probe" für die oben beschriebene ISH-Prozedur I wurde der Einfluss der Formamid-Konzentration in den Hybridisierungslösungen auf die Signalstärke untersucht.

Die Hybridisierungssonde bestand aus grün-markierten Polynukleotiden (Absorption bei 503 nm und Emission bei 528 nm), die gegen die Region 17q11.2-q12 des HER2-Gens gerichtet waren, und orange-markierten Polynukleotiden (Absorption bei 547 nm und Emission bei 572 nm), die gegen die Alpha-Satelliten-Zentromer-Region von Chromosom 17 (D17Z1) gerichtet waren.

Hybridisierungslösung I: 15 Gew.-% Dextransulfat, 500 mM NaCl, 27 Vol.-% Formamid, 1-fach konzentrierter SSC-Puffer, 10,5 Gew.-% Ethylencarbonat, 2 µg/µl Blockier- und Stabilisierungs-DNA und 5 ng/µl lokusspezifische Hybridisierungssonde, jeweils bezogen auf die Zusammensetzung.

Die Hybridisierungslösungen II bis V weisen im Vergleich zu Hybridisierungslösung I verschiedene Formamid-Konzentrationen von 14 Vol.-% (II), 11 Vol.-% (III), 7 Gew.-% (IV) und 0 Vol-% (V) auf.

Die Hybridisierungslösungen I bis V wurden jeweils im Rahmen der oben beschriebenen ISH-Prozedur I sowohl mit kurzen Hybridisierungsdauern von zwei Stunden als auch mit langen Hybridisierungsdauern von 16 Stunden eingesetzt, wobei die Hybridisierungssonde "Flexible HER2/CEN17 Probe" zum Einsatz kam. Die diesbezüglich erhaltenen Signalmuster wurden miteinander verglichen, insbesondere im Hinblick auf die jeweils erzielte Signalstärke.

Mit Hybridisierungslösung I (27 Vol.-% Formamid) wurden sehr starke genspezifische HER2-Signale neben ebenfalls sehr starken CEN17-spezifischen Signalen bei sehr geringem Hintergrund und gut erhaltener Struktur von Zellen und Gewebe sowohl bei den kurzen als auch bei langen Hybridisierungsdauern erhalten.

Geringere Formamid-Konzentrationen in den Hybridisierungslösungen führten nach den zweistündigen Hybridisierungsdauern zu schwächeren oder keinen HER2-spezifischen Signalen. Mit 14 Vol.-% Formamid (Lösung II) wurden starke Signale erzeugt, welche immer noch sehr gut auswertbar waren. Mit 11 Vol.-% Formamid (Lösung III) hingegen wurden nur noch mittelstarke Signale in den Signalmustern erzeugt. Hybridisierungslösungen mit 7 Vol.-% Formamid (Lösungen IV) führten lediglich zu schwachen Signalen, welche keine Auswertung mehr erlaubten. Mit Hybridisierungslösungen ohne Formamid (Lösung V) wurden keine Signale erhalten.

Auch bei langen Hybridisierungsdauern von 16 Stunden führten abnehmende Formamid-Konzentrationen in den Hybridisierungslösungen zu abnehmenden Signalstärken bezüglich der HER2-spezifischen Signale. Mit 14 Vol.-% Formamid (Lösung II) wurden sehr starke Signale erzeugt, welche immer noch sehr gut auswertbar waren. Mit 11 Vol.-% Formamid (Lösung III) wurden ebenfalls noch gut auswertbare starke Signale in den Signalmustern erzeugt. Hybridisierungslösungen mit 7 Vol.-% Formamid (Lösungen IV) führten lediglich zu schwachen Signalen, welche keine Auswertung mehr erlaubten. Mit Hybridisierungslösungen ohne Formamid (Lösung V) wurden keine Signale erhalten.

### Beispiel B.3 Einfluss der Ethylencarbonat-Konzentration auf flexible Schnell-FISH-Verfahren

Unter Einsatz der lokusspezifischen Hybridisierungssonde "Flexible HER2/CEN17 Probe" für die oben beschriebene ISH-Prozedur I wurde der Einfluss der Ethylencarbonat-Konzentration in den Hybridisierungslösungen auf die Signalstärke untersucht.

Die Hybridisierungssonde bestand aus grün-markierten Polynukleotiden (Absorption bei 503 nm und Emission bei 528 nm), die gegen die Region 17q11.2-q12 des HER2-Gens gerichtet waren, und orange-markierten Polynukleotiden (Absorption bei 547 nm und Emission bei 572 nm), die gegen die Alpha-Satelliten-Zentromer-Region von Chromosom 17 (D17Z1) gerichtet waren.

Hybridisierungslösung I: 15 Gew.-% Dextransulfat, 500 mM NaCl, 27 Vol.-% Formamid, 1-fach konzentrierter SSC-Puffer, 10,5 Gew.-% Ethylencarbonat, 2 µg/µl Blockier- und Stabilisierungs-DNA und 5 ng/µl Sonden-DNA, jeweils bezogen auf die Zusammensetzung.

Die Hybridisierungslösungen II bis IV wiesen im Vergleich zu Hybridisierungslösung I andere Ethylencarbonat-Konzentrationen von 7 Gew.-% (II), 5 Gew.-% (III) und 0 Gew.-% (IV) auf.

Die Hybridisierungslösungen I bis IV wurden jeweils im Rahmen der oben beschriebenen ISH-Prozedur I sowohl mit kurzen Hybridisierungsdauern von zwei Stunden als auch mit langen Hybridisierungsdauern von 16 Stunden eingesetzt, wobei die Hybridisierungssonde "Flexible HER2/CEN17 Probe" zum Einsatz kam. Die diesbezüglich erhaltenen Signalmuster wurden miteinander verglichen, insbesondere im Hinblick auf die jeweils erzielte Signalstärke.

Mit Hybridisierungslösung I (10,5 Gew.-% Ethylencarbonat) wurden sehr starke genspezifische HER2-Signale neben ebenfalls sehr starken CEN17-spezifischen Signalen bei sehr geringem Hintergrund und gut erhaltener Struktur von Zellen und Gewebe sowohl mit kurzen als auch mit langen Hybridisierungsdauern erhalten.

Geringere Ethylencarbonat-Konzentrationen in den Hybridisierungslösungen führten nach den zweistündigen Hybridisierungsdauern zu schwächeren oder keinen HER2-spezifischen Signalen. Mit 7 Gew.-% Ethylencarbonat (Lösung II) wurden starke Signale erzeugt, welche immer noch sehr gut auswertbar waren. Mit 5 Gew.-% Ethylencarbonat (Lösung III) hingegen wurden nur noch mittelstarke Signale in den Signalmustern erzeugt. Mit Hybridisierungslösungen ohne Ethylencarbonat (Lösung IV) wurden nur noch sehr schwache und nicht mehr auswertbare Signale erhalten.

Auch bei langen Hybridisierungsdauern von 16 Stunden führten abnehmende Ethylencarbonat-Konzentrationen in den Hybridisierungslösungen zu abnehmenden Signalstärken bezüglich der HER2-spezifischen Signale. Mit 7 Gew.-% Ethylencarbonat (Lösung II) wurden sehr starke Signale erzeugt. Mit 5 Vol.-% Ethylencarbonat (Lösung III) wurden ebenfalls sehr starke Signale in den Signalmustern erzeugt. Mit Hybridisierungslösungen ohne Ethylencarbonat (Lösung IV) wurden nur noch mittelstarke Signale erhalten.

### Beispiel B.4 Einfluss von Dextransulfat auf flexible Schnell-FISH-Verfahren

Unter Einsatz der lokusspezifischen Hybridisierungssonde "Flexible HER2/CEN17 Probe" für die oben beschriebene ISH-Prozedur I wurde der Einfluss von Dextransulfat in den Hybridisierungslösungen auf die Signalstärke untersucht. Im Zusammenhang mit der Durchführung der Hybridisierung sowie der Spezifität der Sonden wurde auf obige Ausführungen verwiesen.

Hybridisierungslösung I: 15 Gew.-% Dextransulfat, 500 mM NaCl, 27 Vol.-% Formamid, 1-fach konzentrierter SSC-Puffer, 10,5 Gew.-% Ethylencarbonat, 2 µg/µl Blockier- und Stabilisierungs-DNA und 5 ng/µl Sonden-DNA, jeweils bezogen auf die Zusammensetzung.

Hybridisierungslösung II: 500 mM NaCl, 27 Vol.-% Formamid, 1-fach konzentrierter SSC-Puffer, 10,5 Gew.-% Ethylencarbonat, 2 µg/µl Blockier- und Stabilisierungs-DNA und 5 ng/µl Sonden-DNA, jeweils bezogen auf die Zusammensetzung.

Die mit den Hybridisierungslösungen I bzw. II erhaltenen Signalmuster wurden miteinander verglichen, insbesondere im Hinblick auf die jeweils erzielte Signalstärke.

In den mit Hybridisierungslösung I erhaltenen Signalmustern zeigten sich sehr starke genspezifische HER2-Signale neben ebenfalls sehr starken CEN17-spezifischen Signalen bei sehr geringem Hintergrund und gut erhaltener Struktur der Zellen und Gewebe sowohl mit kurzen als auch mit langen Hybridisierungsdauern. Die Hybridisierungslösung II ohne Dextransulfat führte bei kurzen Hybridisierungsdauern zu keinen sichtbaren bzw. detektierbaren Signalen. Bei langen Hybridisierungsdauern wurden mit Hybridisierungslösung II lediglich sehr schwache bzw. keine sichtbaren bzw. detektierbaren Signale erhalten.

### Beispiel B.5 Einfluss verschiedener polarer protischer bzw. polarer aprotischer Lösemittel mit cyclischer Molekülstruktur auf flexible Schnell-FISH-Verfahren

Unter Einsatz der lokusspezifischen Hybridisierungssonde "Flexible HER2/CEN17 Probe" für die oben beschriebene ISH-Prozedur I wurde der Einfluss verschiedener polarer protischer bzw. polarer aprotischer Lösemittel mit cyclischer Molekülstruktur in den Hybridisierungslösungen auf die Signalstärke untersucht. Im Zusammenhang mit der Durchführung der Hybridisierung sowie die Spezifität der Sonden wird auf obige Ausführungen verwiesen.

Hybridisierungslösung I: 15 Gew.-% Dextransulfat, 500 mM NaCl, 27 Vol.-% Formamid, 1-fach konzentrierter SSC-Puffer, 10,5 Gew.-% Ethylencarbonat (I), 2 µg/µl Blockier- und Stabilisierungs-DNA und 5 ng/µl Sonden-DNA, jeweils bezogen auf die Zusammensetzung.
Hybridisierungslösung II: anstelle von Ethylencarbonat 10,5 Gew.-% 2-Piperidon (Valerolactam)
Hybridisierungslösung III: anstelle von Ethylencarbonat 10,5 Vol.-% 2-Pyrrolidon (γ-Butyrolactam)
Hybridisierungslösung IV: anstelle von Ethylencarbonat 10,5 Gew.-% 3-Sulfolen (Butadien-Sulfon)
Hybridisierungslösung V: anstelle von Ethylencarbonat 10,5 Vol.-% γ-Butyrolacton

Die mit den Hybridisierungslösungen I bis V erhaltenen Signalmuster wurden miteinander verglichen, insbesondere im Hinblick auf die jeweils erzielte Signalstärke.

Mit allen Hybridisierungslösungen I bis V, d.h. mit allen getesteten polaren protischen bzw. polaren aprotischen Lösemitteln mit cyclischer Molekülstruktur, wurden sehr starke genspezifische HER2-Signale neben ebenfalls sehr starken CEN17-spezifischen Signalen bei sehr geringem Hintergrund und gut erhaltener Struktur von Zellen und Gewebe sowohl mit kurzen als auch mit langen Hybridisierungsdauern erhalten. Die besten Ergebnisse wurden mit 2-Pyrrolidon (γ-Butyrolactam), γ-Butyrolacton und Ethylencarbonat erzielt, wobei jedoch auch mit 2-Piperidon (Valerolactam) und 3-Sulfolen ("Butadiene Sulfone") sehr gute Ergebnisse erhalten wurden.

Diese Ergebnisse wurden sowohl bei normalen Zellen/Geweben bzw. Zellen/Geweben ohne Aberrationen an Chromosom 17 als auch bei Zellen/Geweben, insbesondere aus Mammakarzinomen, mit HER2-Amplifikationen beobachtet. Somit konnten auch mit allen jeweils einzeln verwendeten polaren protischen bzw. polaren aprotischen Lösemitteln mit cyclischer Molekülstruktur die zu identifizierenden HER2-Amplifikationen eindeutig identifiziert werden.

### Beispiel B.6 FISH zum Nachweis von genspezifischen ALK-Signalen in automatisierten FISH-Verfahren

Zum Nachweis von ALK-spezifischen Signalen in automatisierten FISH-Verfahren wurden Hybridisierungslösungen auf Basis von Formamid und Ethylencarbonat eingesetzt, welche als lokusspezifische Hybridisierungssonde "Auto ALK Break Apart Probe" enthielten. Die FISH also solche wurde gemäß der oben beschriebenen ISH-Prozedur II durchgeführt (automatisierte FISH auf dem Automaten Celerus Wave RPD System).

Die lokusspezifische Hybridisierungssonde bestand aus grün-markierten Polynukleotiden (Absorption bei 503 nm und Emission bei 528 nm), die in 2p23 gegen proximal zur ALK-Bruchpunktregion gelegenen Sequenzen gerichtet waren, und orange-markierten Polynukleotiden (Absorption bei 547 nm und Emission bei 572 nm), die in 2p23 gegen distal zur ALK-Bruchpunktregion gelegenen Sequenzen gerichtet waren.

Hybridisierungslösung: 18 Gew.-% Dextransulfat, 600 mM NaCl, 22 Vol.-% Formamid, 1-fach konzentrierter SSC-Puffer, 12,75 Gew.-% Ethylencarbonat, 0,1 µg/µl Blockier- und Stabilisierungs-DNA und 2 ng/µl lokusspezifische Hybridisierungssonde, jeweils bezogen auf die Zusammensetzung.

In dem erhaltenen Signalmuster zeigten sich sehr starke ALK-spezifische Signale bei sehr geringem Hintergrund und gut erhaltener Struktur der Zellen und Gewebe. Dies konnte sowohl bei normalen Zellen/Geweben bzw. Zellen/Geweben ohne Aberrationen an Chromosom 2 als auch bei Zellen/Geweben mit ALK-Aberrationen beobachtet werden. Somit konnten die mit der Hybridisierungssonde zu identifizierenden ALK-Aberrationen (d.h. Break-Apart-Ereignisse von grün-orangenen Fusionssignalen) eindeutig identifiziert werden.

### Beispiel B.7 Einfluss der Formamid-Konzentration auf automatisierte FISH-Verfahren

Unter Einsatz der lokusspezifischen Hybridisierungssonde "Auto HER2/CEN17 Probe" für die oben beschriebene ISH-Prozedur III wurde der Einfluss der Formamid-Konzentration in den Hybridisierungslösungen auf die Signalstärke untersucht.

Die lokusspezifische Hybridisierungssonde bestand aus orange-markierten Polynukleotiden (Absorption bei 547 nm und Emission bei 572 nm), die gegen die Region 17q11.2-q12 des HER2-Gens gerichtet waren, und grün-markierten Polynukleotiden (Absorption bei 503 nm und Emission bei 528 nm), die gegen die Alpha-Satelliten-Zentromer-Region von Chromosom 17 (D17Z1) gerichtet waren.

Hybridisierungslösung I: 18 Gew.-% Dextransulfat, 600 mM NaCl, 21 Vol.-% Formamid, 1-fach konzentrierter SSC-Puffer, 12,75% Gew.-% Ethylencarbonat, 0,1 µg/µl Blockier- und Stabilisierungs-DNA und 2 ng/µl Hybridisierungssonde, jeweils bezogen auf die Zusammensetzung.

Die Hybridisierungslösungen II bis V wiesen im Vergleich zu Hybridisierungslösung I unterschiedliche Formamid-Konzentrationen von 17 Vol.-% (II), 13 Vol.-% (III), 9 Vol.-% (IV) und 0 Vol.-% (V) auf.

Mit Hybridisierungslösung I wurden sehr starke genspezifische HER2-Signale (neben ebenfalls sehr starken CEN17-spezifischen Signalen) bei sehr geringem Hintergrund und gut erhaltener Struktur der Zellen und Gewebe detektiert. Auch mit Hybridisierungslösung II (17 Vol.-% Formamid) wurden sehr starke Signale erhalten. Hybridisierungslösung III mit 13 Vol.-% Formamid führte noch zu starken Signalen, wohingegen mit 9 Vol.-% (Hybridisierungslösung IV) nur noch mittelstarke Signale erhalten wurden. Enthielten die Hybridisierungslösungen (Hybridisierungslösung V) kein Formamid, wurden keine Signale erhalten.

### Beispiel B.8 Einfluss der Ethylencarbonat-Konzentration auf automatisierte FISH-Verfahren

Unter Einsatz der lokusspezifischen Hybridisierungssonde "Auto HER2/CEN17 Probe" für die oben beschriebene ISH-Prozedur III wurde der Einfluss der Ethylencarbonat-Konzentration in den Hybridisierungslösungen auf die Signalstärke untersucht. Im Zusammenhang mit der Durchführung der Hybridisierung und der Spezifität der Sonden wurde auf obige Ausführungen im Zusammenhang mit der Hybridisierungssonde "Auto HER2/CEN17 Probe" verwiesen.

Hybridisierungslösung I: 18 Gew.-% Dextransulfat, 600 mM NaCl, 21 Vol.-% Formamid, 1-fach konzentrierter SSC-Puffer, 12,75 % Gew.-% Ethylencarbonat, 0,1 µg/µl Blockier- und Stabilisierungs-DNA und 2 ng/µl Hybridisierungssonde, jeweils bezogen auf die Zusammensetzung.

Die Hybridisierungslösungen II bis IV wiesen im Vergleich zu Hybridisierungslösung I unterschiedliche Ethylencarbonat-Konzentrationen von 9 Gew.-% (II), 6 Gew.-% (III) und 0 Gew.-% (IV) auf.

Mit Hybridisierungslösung I wurden sehr starke genspezifische HER2-Signale (neben ebenfalls sehr starken CEN17-spezifischen Signalen) bei sehr geringem Hintergrund und gut erhaltener Struktur der Zellen und Gewebe detektiert. Mit Hybridisierungslösung II (9 Gew.-% Ethylencarbonat) wurden bereits nur noch mittelstarke Signale erhalten. Hybridisierungslösungen III und IV mit 6 Vol.-% Ethylencarbonat bzw. ohne Ethylencarbonat führten nur noch zu schwachen und nicht mehr auswertbaren Signalen.

### Beispiel B.9 Einfluss von Dextransulfat auf automatisierte FISH-Verfahren

Unter Einsatz der lokusspezifischen Hybridisierungssonde "Auto ROS1 Break Apart Probe" für die oben beschriebene ISH-Prozedur II (automatisierte FISH auf dem Automaten Celerus Wave RPD System) wurde der Einfluss von Dextransulfat in den Hybridisierungslösungen auf die Signalstärke untersucht. Im Zusammenhang mit der Durchführung der Hybridisierung wird auf obige Ausführungen bezüglich Prozedur II verwiesen.

Die Hybridisierungssonde bestand aus grün-markierten Polynukleotiden (Absorption bei 503 nm und Emission bei 528 nm), die in 6q22 gegen proximal zur ROS1-Bruchpunktregion gelegene Sequenzen gerichtet waren, und orange-markierten Polynukleotiden (Absorption bei 547 nm und Emission bei 572 nm), die in 6q22 gegen distal zur ROS1-Bruchpunktregion gelegene Sequenzen gerichtet waren.

Hybridisierungslösung I: 18 Gew.-% Dextransulfat, 600 mM NaCl, 22 Vol.-% Formamid, 1-fach konzentrierter SSC-Puffer, 12,75 Gew.-% Ethylencarbonat, 0,1 µg/µl Blockier- und Stabilisierungs-DNA und 2 ng/µl lokusspezifische Hybridisierungssonden, jeweils bezogen auf die Zusammensetzung.

Hybridisierungslösung II enthielt im Vergleich zu Hybridisierungslösung I kein Dextransulfat.

Mit Hybridisierungslösung I wurden sehr starke genspezifische ROS1-Signale bei sehr geringem Hintergrund und gut erhaltener Struktur von Zellen und Gewebe erzielt. Mit Hybridisierungslösung II, welche die Hybridisierungssonde in einer Zusammensetzung ohne Dextransulfat enthält, wurden keine sichtbaren bzw. detektierbaren Signale erzielt.

### Beispiel B.10 Einfluss verschiedener ausgewählter polarer protischer bzw. polarer aprotischer Lösemittel mit cyclischer Molekülstruktur auf automatisierte FISH-Verfahren

Unter Einsatz der lokusspezifischen Hybridisierungssonde "Auto HER2/CEN17 Probe" für die oben beschriebene ISH-Prozedur III (automatisierte FISH auf dem Automaten Pathcom Stainer) wurde der Einfluss von verschiedenen polaren protischen bzw. polaren aprotischen Lösemitteln mit cyclischer Struktur in den Hybridisierungslösungen auf die Signalstärke untersucht. Es wurde die ISH-Prozedur III (vgl. obige Ausführungen zu ISH-Prozedur III) unter Einsatz des Automaten Pathcom Stainer durchgeführt. In Bezug auf Spezifität und Markierung der Hybridisierungssonde "Auto HER2/CEN17 Probe" wird zur Vermeidung unnötiger Wiederholungen auf obige Ausführungen verwiesen.

Hybridisierungslösungen I bis V: 18 Gew.-% Dextransulfat, 600 mM NaCl, 22 Vol.-% Formamid, 1-fach konzentrierter SSC-Puffer, 12,75 Gew.-% bzw. Vol.-% (je nach eingesetzter Substanz) eines polaren protischen bzw. polaren aprotischen Lösemittels mit cyclischer Struktur, 0,1 µg/µl Blockier- und Stabilisierungs-DNA und 2 ng/µl Hybridisierungssonde, jeweils bezogen auf die Zusammensetzung, wobei als polares protisches bzw. polares aprotisches Lösemittel mit cyclischer Struktur entweder Ethylencarbonat (I, Gew.-%), 2-Piperidon (Valerolactam) (II, Gew.-%), 2-Pyrrolidon (γ-Butyrolactam) (III, Vol.-%), 3-Sulfolen ("Butadiene Sulfone") (IV, Gew.-%) und γ-Butyrolacton (V, Vol.-%) eingesetzt wurden.

Mit allen untersuchten polaren protischen bzw. polaren aprotischen Lösemitteln mit cyclischer Struktur wurden starke bis sehr starke genspezifische HER2-Signale (neben ebenfalls starken bis sehr starken CEN17-spezifischen Signalen) bei sehr geringem Hintergrund und gut erhaltener Struktur der Zellen und Gewebe im Rahmen der In-Situ-Hybridisierungen erhalten. Es zeigten sich nur geringfügige Unterschiede bezüglich der Intensität der Signale in folgender Abstufung von sehr stark zu jeweils geringfügig schwächer: γ-Butyrolacton, 2-Pyrrolidon (γ-Butyrolactam), Ethylencarbonat, 2-Piperidon (Valerolactam) und 3-Sulfolen ("Butadiene Sulfone"). Diese Ergebnisse konnten sowohl bei normalen Zellen/Geweben bzw. Zellen/Geweben ohne Aberrationen an Chromosom 17 als auch bei Zellen/Geweben, insbesondere Mammakarzinomen, mit HER2-Amplifikationen beobachtet werden. Somit konnten auch mit allen jeweils einzeln verwendeten polaren protischen bzw. polaren aprotischen Lösemitteln mit cyclischer Struktur die zu detektierenden HER2-Amplifikationen (d.h. entweder mehrere HER2-spezifische Signale oder HER2-Signal-Cluster) eindeutig identifiziert werden.

### Beispiel B.11 Einfluss der Bewegung auf die Hybridisierung in automatisierten FISH-Verfahren

Unter Einsatz der lokusspezifischen Hybridisierungssonde "Auto HER2/CEN17 Probe" für die oben beschriebene ISH-Prozedur III wurde der Einfluss insbesondere wellenförmiger Bewegungen während des Hybridisierungs-Schrittes von automatisierten In-Situ-Hybridisierungen auf die resultierenden Signalmuster untersucht. Dazu wurde die oben beschriebene ISH-Prozedur III auf dem Automaten "Pathcom Stainer" unter Verwendung der Hybridisierungssonde "Auto HER2/CEN17 Probe" durchgeführt.

Im Zusammenhang mit der Spezifität der Sonden wird auf obige Ausführungen im Zusammenhang mit der Hybridisierungssonde "Auto HER2/CEN17 Probe" verwiesen.

Die Hybridisierungslösungen enthielten 18 Gew.-% Dextransulfat, 600 mM NaCl, 21 Vol.-% Formamid, 1-fach konzentrierten SSC-Puffer, 12,75 Gew.-% Ethylencarbonat, 0,1 µg/µl Blockier- und Stabilisierungs-DNA und 2 ng/µl lokusspezifische Hybridisierungssonden.

Zu Vergleichszwecken wurde die ISH-Prozedur III unter Bewegung, insbesondere wellenförmiger Bewegung, von Proben und Hybridisierungszusammensetzung bzw. -lösung und ohne Bewegung von Proben und Hybridisierungszusammensetzung bzw. -lösung während der Hybridisierung durchgeführt. Die Hybridisierung erfolgte jeweils für 120 Minuten bei 45 °C.

Die Bewegung der Proben bzw. Hybridisierungslösungen erfolgte kontinuierlich durch Bewegen der Reaktionskammer des Automaten. Die Bewegung der Reaktionskammer wurde in Intervallen von 15 Sekunden, zwei Minuten bzw. 10 Minuten mit einer Öffnungszeit der Abdeckung der Hybridisierungskammer von jeweils einer Sekunde induziert. Zu Vergleichszwecken wurde die Hybridisierung der Proben ohne Bewegen bzw. ohne Öffnen der Hybridisierungskammer durchgeführt.

Mit allen Ansätzen, welche unter Bewegung bzw. unter Öffnen der Hybridisierungskammer durchgeführt wurden, wurden sehr starke genspezifische HER2-Signale (neben ebenfalls sehr starken CEN17-spezifischen Signalen) bei sehr geringem Hintergrund und gut erhaltener Morphologie von Zellen und Geweben erhalten. Die hervorragenden Ergebnisse wurden gleichermaßen für Bewegungsintervalle von 15 Sekunden, zwei Minuten und 10 Minuten erhalten. Ohne Bewegung von Hybridisierungslösungen bzw. Proben wurden ebenfalls gut auswertbare Signale bzw. Signalmuster erhalten, allerdings fielen die Signale schwächer aus als bei den Hybridisierungen, welche unter Bewegung von Hybridisierungslösungen bzw. Proben durchgeführt wurden.

### Beispiel B.12 Einfluss von Detergenzien auf die Hybridisierung in automatisierten FISH-Verfahren

Unter Einsatz der lokusspezifischen Hybridisierungssonde "Auto ROS1 Break Apart Probe" für die oben beschriebene ISH-Prozedur III (automatisierte FISH auf dem Automaten Pathcom Stainer) wurde der Einfluss des Polyalkylenglycolethers Brij^{®}-35 (synonym auch als Brij^{®}-L23 oder Polyoxyethylen(23)laurylether bezeichnet) in den Hybridisierungslösungen auf die Signalstärke untersucht. Im Zusammenhang mit der Durchführung der Hybridisierung gemäß ISH-Prozedur III sowie der Spezifität der Hybridisierungssonde "Auto ROS1 Break Apart Probe" wird auf obige Ausführungen verwiesen.

Hybridisierungslösung I: 18 Gew.-% Dextransulfat, 600 mM NaCl, 22 Vol.-% Formamid, 1-fach konzentrierter SSC-Puffer, 9,95 Gew.-% Ethylencarbonat, 0,1 µg/µl Blockier- und Stabilisierungs-DNA und 2 ng/µl lokusspezifische Hybridisierungssonden, jeweils bezogen auf die Zusammensetzung.

Die Hybridisierungslösungen II bis VI wiesen im Vergleich zu Hybridisierungslösung I andere Brij^{®}-35-Konzentrationen von 0,125 Gew.-% (II), 0,2 Gew.-% (III), 0,4 Gew.-% (IV), 0,9 Gew.-% (V) und 1,8 Gew.-% (VI) auf, jeweils bezogen auf die Zusammensetzung.

Mit Hybridisierungslösung I (ohne Brij^{®}-35) wurden starke genspezifische ROS1-Signale bei geringem Hintergrund und gut erhaltener Struktur von Zellen und Gewebe erzielt.

Mit den Hybridisierungslösungen II bis V (Brij^{®}-35-Konzentrationen von 0,125 Gew.-% bis 0,9 Gew.-%) wurden sehr starke Signale erhalten. Diese im Vergleich zu Hybridisierungslösung I (ohne Brij^{®}-35) stärkeren Signale könnten - ohne sich hierbei auf diese Theorie beschränken zu wollen - auf eine mit dem Einsatz des Detergens einhergehende schwächere Kernfärbung und somit besseren Kontrast (Signal zu Hintergrund) zurückzuführen sein.

Enthalten die Hybridisierungslösungen das Detergens Brij^{®}-35 in einer Konzentration von 1,8 Gew.-% (Hybridisierungslösung VI), bezogen auf die Zusammensetzung, wurden nur noch mittelstarke Signale erhalten.

### Bevorzugte Ausführungsformen

In erfindungsgemäß bevorzugten Ausführungsformen, betrifft die Erfindung:
1. Eine Zusammensetzung, insbesondere Zusammensetzung zur Verwendung bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en), wobei die Zusammensetzung enthält:
   (a) mindestens eine, vorzugsweise lokusspezifische, Hybridisierungssonde ("Komponente (a)");
   (b) mindestens ein polares protisches oder polares aprotisches Lösemittel ("Komponente (b)"), vorzugsweise mit cyclischer Molekülstruktur; und
   (c) mindestens ein Carbonsäureamid und/oder dessen Salze ("Komponente (c)"), insbesondere Formamid und/oder dessen Salze, in einer Menge von mehr als 10 Vol.-%, bezogen auf die Zusammensetzung.
2. Zusammensetzung nach Ausführungsform 1,
   wobei die Zusammensetzung (a) die mindestens eine, vorzugsweise lokusspezifische, Hybridisierungssonde in einer Konzentration im Bereich von 0,1 ng/µl bis 50 ng/µl, insbesondere 0,5 ng/µl bis 50 ng/µl, vorzugsweise 0,7 ng/µl bis 8 ng/µl, bevorzugt 1 ng/µl bis 5 ng/µl, bezogen auf die Zusammensetzung, enthält; und/oder
   wobei (b) das mindestens eine polare protische oder polare aprotische Lösemittel ausgewählt ist aus der Gruppe von Lösemitteln mit Lacton-, Sulfon-, Nitril-, Carbonat- und/oder Amid-Funktionalität, und/oder
   wobei (b) das mindestens eine polare aprotische oder polares protische Lösemittel ausgewählt ist aus der Gruppe von Ethylencarbonat, Pyrrolidonen, Lactamen, Ethylensulfit, γ-Butyrolacton, Ethylentrithiocarbonat, Propylencarbonat und/oder Sulfolan, insbesondere Ethylencarbonat und/oder Pyrrolidonen, bevorzugt Ethylencarbonat, und/oder
   wobei (b) das mindestens eine Lösemittel, vorzugsweise mit cyclischer Molekülstruktur, ein polares aprotisches Lösemittel ist, insbesondere ausgewählt aus cyclischen Carbonaten (cyclischen Kohlensäurestern), insbesondere cyclischen Kohlensäureestern von Alkylenglykolen, bevorzugt Ethylencarbonat (1,3-Dioxolan-2-on) und Propylencarbonat, besonders bevorzugt Ethylencarbonat, sowie cyclischen Mono-, Di- und Trithiocarbonaten, insbesondere Ethylensulfit und Ethylentrithiocarbonat, und/oder
   wobei (b) das mindestens eine Lösemittel, vorzugsweise mit cyclischer Molekülstruktur, ein polares aprotisches Lösemittel ist, insbesondere ausgewählt aus aprotischen cyclischen Amiden (Lactamen), insbesondere N-alkylsubstituierten Pyrrolidonen, vorzugsweise N-Methyl-2-pyrrolidon und/oder N-Ethyl-2-pyrrolidon, und/oder
   wobei (b) das mindestens eine Lösemittel, vorzugsweise mit cyclischer Molekülstruktur, ein polares protisches Lösemittel ist, insbesondere ausgewählt aus protischen cyclischen Amiden (Lactamen), insbesondere protischen cyclischen Amiden (Lactamen) mit einem Wasserstoffatom am Amid-Stickstoff (Lactam-Stickstoff), bevorzugt aus der Gruppe von 2-Pyrrolidon (γ-Butyrolactam), 3-Pyrrolidon, Caprolactamen und/oder 2-Piperidon (Valerolactam), besonders bevorzugt Pyrrolidonen, ganz besonders bevorzugt 2-Pyrrolidon (γ-Butyrolactam), und/oder
   wobei (b) das mindestens eine Lösemittel ein polares protisches Lösemittel mit cyclischer Molekülstruktur ist, wobei die cyclische Molekülstruktur einen N-Heterocyclus mit freiem Wasserstoffatom am Stickstoffatom umfasst, und/oder
   wobei (b) das mindestens eine polare protische oder polare aprotische Lösemittel, vorzugsweise mit cyclischer Molekülstruktur, ausgewählt ist aus der Gruppe von γ-Butyrolacton, 2-Pyrrolidon (γ-Butyrolactam) und/oder Ethylencarbonat.
3. Zusammensetzung nach Ausführungsform 1 oder 2,
   wobei die Zusammensetzung (b) das mindestens eine polare protische oder polare aprotische Lösemittel, vorzugsweise mit cyclischer Molekülstruktur, in einer Menge enthält, welche nicht zur Denaturierung von Nukleinsäuren führt, und/oder wobei die Zusammensetzung (b) das mindestens eine polare protische oder polare aprotische Lösemittel, vorzugsweise mit cyclischer Molekülstruktur, in einer Menge im Bereich von 0,5 bis 40 Vol.-% oder Gew.-%, insbesondere 1 bis 35 Vol.-% oder Gew.-%, vorzugsweise 2 bis 30 Vol.-% oder Gew.-%, bevorzugt 5 bis 20 Vol.-% oder Gew.-%, besonders bevorzugt 7 bis 15 Vol.-% oder Gew.-%, bezogen auf die Zusammensetzung, enthält; und/oder
   wobei die Zusammensetzung (b) das mindestens eine polare protische oder polare aprotische Lösemittel, vorzugsweise mit cyclischer Molekülstruktur, in einer Menge von mindestens 0,5 Vol.-% oder Gew.-%, insbesondere mindestens 1 Vol.-% oder Gew.-%, vorzugsweise mindestens 2 Vol.-% oder Gew.-%, bevorzugt mindestens 5 Vol.-% oder Gew.-%, besonders bevorzugt mindestens 7 Vol.-% oder Gew.-%, ganz besonders bevorzugt mindestens 10 Vol.-% oder Gew.-%, enthält und/oder wobei die Zusammensetzung (b) das mindestens eine polare protische oder polare aprotische Lösemittel, vorzugsweise mit cyclischer Molekülstruktur, in einer Menge von höchstens 50 Vol.-% oder Gew.-%, insbesondere höchstens 40 Vol.-% oder Gew.-%, vorzugsweise höchstens 30 Vol.-% oder Gew.-%, bevorzugt höchstens 20 Vol.-% oder Gew.-%, besonders bevorzugt höchstens 15 Vol.-% oder Gew.-%, ganz besonders bevorzugt höchstens 13 Vol.-% oder Gew.-%, bezogen auf die Zusammensetzung, enthält.
4. Zusammensetzung nach einer der vorangehenden Ausführungsformen,
   wobei die Zusammensetzung (c) das mindestens eine Carbonsäureamid und/oder dessen Salze, insbesondere Formamid und/oder dessen Salze, in einer Menge enthält, welche zur Denaturierung von Nukleinsäuren führt und/oder wobei die Zusammensetzung (c) das mindestens eine Carbonsäureamid und/oder dessen Salze, insbesondere Formamid und/oder dessen Salze, in einer Menge im Bereich von 10 bis 60 Vol.-%, insbesondere 15 bis 50 Vol.-%, vorzugsweise 17 bis 40 Vol.-%, bevorzugt 20 bis 30 Vol.-%, bezogen auf die Zusammensetzung, enthält; und/oder
   wobei die Zusammensetzung (c) das mindestens eine Carbonsäureamid und/oder dessen Salze, insbesondere Formamid und/oder dessen Salze, in einer Menge von mindestens 10 Vol.-%, insbesondere mindestens 15 Vol.-%, vorzugsweise mindestens 17 Vol.-%, bevorzugt mindestens 20 Vol.-%, bezogen auf die Zusammensetzung, enthält und/oder wobei die Zusammensetzung (c) das mindestens eine Carbonsäureamid und/oder dessen Salze, insbesondere Formamid und/oder dessen Salze, in einer Menge von höchstens 60 Vol.-%, insbesondere höchstens 50 Vol.-%, vorzugsweise höchstens 40 Vol.-%, bevorzugt höchstens 30 Vol.-%, bezogen auf die Zusammensetzung, enthält; und/oder
   wobei die Zusammensetzung die Komponente (b) und die Komponente (c) bzw. das mindestens eine polare protische oder polare aprotische Lösemittel, vorzugsweise mit cyclischer Molekülstruktur, und das mindestens eine Carbonsäureamid und/oder dessen Salze, insbesondere Formamid und/oder dessen Salze, in einem volumenbezogenen Verhältnis im Bereich von 1 : 100 bis 50 : 1, insbesondere 1 : 50 bis 20 : 1, vorzugsweise 1 : 25 bis 10 : 1, bevorzugt 1 : 10 bis 5 : 1, besonders bevorzugt 1 : 5 bis 1 : 1, ganz besonders bevorzugt 1 : 3 bis 1 : 2 , enthält.
5. Zusammensetzung nach einer der vorangehenden Ausführungsformen,
   wobei die Zusammensetzung mindestens ein Polysaccharid ("Komponente (d)"), insbesondere ein Biopolysaccharid, vorzugsweise ein neutrales Biopolysaccharid, bevorzugt Dextran und/oder dessen Derivate oder Salze, besonders bevorzugt Dextransulfat, enthält, insbesondere wobei die Zusammensetzung die Komponente (d) in einer Menge im Bereich von 0,1 bis 50 Gew.-%, insbesondere 1 bis 40 Gew.-%, vorzugsweise 5 bis 30 Gew.-%, bevorzugt 10 bis 20 Gew.-%, besonders bevorzugt 13 bis 18 Gew.-%, bezogen auf die Zusammensetzung, enthält.
6. Zusammensetzung nach einer der vorangehenden Ausführungsformen,
   wobei die Zusammensetzung mindestens ein chemisches Puffersystem, insbesondere in Form von Puffersalz(en) ("Komponente (e)"), enthält,
   insbesondere wobei das chemische Puffersystem zur Einstellung und/oder Konstanthaltung des pH-Werts der Zusammensetzung dient, und/oder
   insbesondere wobei die Zusammensetzung (e) das chemische Puffersystem, bezogen auf die Zusammensetzung und berechnet als Summe aller Bestandteile des chemischen Puffersystems, in einer Menge im Bereich von 0,001 bis 5 Gew.-%, insbesondere 0,01 bis 4 Gew.-%, vorzugsweise 0,1 bis 3 Gew.-%, bevorzugt 0,5 bis 2 Gew.-%, besonders bevorzugt 1 bis 1,5 Gew.-%, enthält, und/oder
   insbesondere wobei das chemische Puffersystem mindestens ein Salz enthält, insbesondere mindestens ein Carbonsäuresalz, vorzugsweise ein Citrat, und/oder mindestens ein anorganisches Salz, insbesondere mindestens ein Alkali- und/oder Erdalkali-Salz, vorzugsweise mindestens ein Alkali- und/oder Erdalkali-Chlorid, besonders bevorzugt Natriumchlorid, und/oder
   wobei das chemische Puffersystem ein citratbasiertes Puffersystem ist und/oder ein citratbasiertes Puffersystem auf der Grundlage von tri-Natriumcitrat/Natriumchlorid ist; und/oder wobei die Zusammensetzung einen pH-Wert im Bereich von 5,0 bis 9,0, insbesondere im Bereich von 5,5 bis 8,5, vorzugsweise im Bereich von 6,0 bis 8,0, bevorzugt im Bereich von 6,5 bis 7,5, aufweist.
7. Zusammensetzung nach einer der vorangehenden Ausführungsformen,
   wobei die Zusammensetzung mindestens ein Blockier- und/oder Stabilisierungsmittel ("Komponente (f)") enthält, insbesondere wobei das Blockier- und/oder Stabilisierungsmittel auf Nukleinsäuren basiert, vorzugsweise auf DNA und/oder RNA, insbesondere wobei die Zusammensetzung (f) das mindestens eine Blockier- und/oder Stabilisierungsmittel in einer Konzentration im Bereich von 0,001 µg/µl bis 100 µg/µl, insbesondere 0,005 µg/µl bis 80 µg/µl, vorzugsweise 0,01 µg/µl bis 40 µg/µl, bevorzugt 0,05 µg/µl bis 20 µg/µl, besonders bevorzugt 0,1 µg/µl bis 10 µg/µl, bezogen auf die Zusammensetzung, enthält; und/oder
   wobei die Zusammensetzung mindestens ein anorganisches Salz ("Komponente (g)"), insbesondere Alkali- und/oder Erdalkali-Salz, vorzugsweise Alkali- und/oder Erdalkali-Chlorid, besonders bevorzugt Natriumchlorid, enthält, insbesondere wobei die Zusammensetzung (g) das mindestens eine anorganische Salz in einer Menge im Bereich von 0,01 bis 15 Gew.-%, insbesondere 0,05 bis 10 Gew.-%, vorzugsweise 0,1 bis 10 Gew.-%, bevorzugt 0,5 bis 5 Gew.-%, besonders bevorzugt 1 bis 3 Gew.-%, bezogen auf die Zusammensetzung, enthält.
8. Zusammensetzung nach einer der vorangehenden Ausführungsformen,
   wobei die Zusammensetzung mindestens ein Detergens und/oder Tensid ("Komponente (h)") enthält, insbesondere wobei das Detergens und/oder Tensid ausgewählt ist aus nichtionischen Tensiden, vorzugsweise Polyalkylenglycolethern, besonders bevorzugt Polyalkylenglycolethern des Laurylalkohols und/oder des Cetylalkohols und/oder des Cetylstearylalkohols und/oder des Oleylalkohols, insbesondere des Laurylalkohols, und/oder insbesondere wobei das Detergens und/oder Tensid ausgewählt ist aus Polyoxyethylen(4)laurylether, Polyoxyethylen(9)laurylether und/oder Polyoxyethylen(23)laurylether, insbesondere Polyoxyethylen(23)laurylether, und/oder insbesondere wobei die Zusammensetzung das mindestens eine Detergens und/oder Tensid in einer Menge im Bereich von 0,001 bis 5 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,12 bis 1 Gew.-%, bezogen auf die Zusammensetzung, aufweist; und/oder
   wobei die Zusammensetzung Wasser, insbesondere gereinigtes Wasser, in einer Menge im Bereich von 10 bis 99 Gew.-%, insbesondere im Bereich von 20 bis 95 Gew.-%, bevorzugt im Bereich von 30 bis 90 Gew.-%, besonders bevorzugt im Bereich von 40 bis 85 Gew.-%, bezogen auf die Zusammensetzung, enthält und/oder wobei die Zusammensetzung Wasser als Träger (Exzipienten) enthält und/oder wobei die Zusammensetzung wässrig basiert ausgebildet ist und/oder wobei die Zusammensetzung Wasser in einer Menge enthält, dass in der Summe unter Einbeziehung aller Komponenten stets 100 % bzw. 100 Gew.-% resultieren, bezogen auf die Zusammensetzung.
9. Zusammensetzung, insbesondere Zusammensetzung zur Verwendung bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en), vorzugsweise Zusammensetzung nach einer der vorangehenden Ausführungsformen,
   wobei die Zusammensetzung enthält:
   (a) mindestens eine, vorzugsweise lokusspezifische, Hybridisierungssonde ("Komponente (a)"), insbesondere in einer Konzentration im Bereich von 0,1 ng/µl bis 50 ng/µl, insbesondere 0,5 ng/µl bis 50 ng/µl, vorzugsweise 0,7 ng/µl bis 8 ng/µl, bevorzugt 1 ng/µl bis 5 ng/µl, bezogen auf die Zusammensetzung;
   (b) mindestens ein polares protisches oder polares aprotisches Lösemittel ("Komponente (b)"), vorzugsweise mit cyclischer Molekülstruktur, insbesondere in einer Menge im Bereich von 0,5 bis 40 Vol.-%, insbesondere 1 bis 35 Vol.-%, vorzugsweise 2 bis 30 Vol.-%, bevorzugt 5 bis 20 Vol.-%, besonders bevorzugt 7 bis 15 Vol.-%, bezogen auf die Zusammensetzung;
   (c) mindestens ein Carbonsäureamid und/oder dessen Salze ("Komponente (c)"), insbesondere Formamid und/oder dessen Salze, insbesondere in einer Menge im Bereich von 10 bis 60 Vol.-%, insbesondere 15 bis 50 Vol.-%, vorzugsweise 17 bis 40 Vol.-%, bevorzugt 20 bis 30 Vol.-%, bezogen auf die Zusammensetzung;
   (d) gegebenenfalls mindestens ein Polysaccharid ("Komponente (d)"), vorzugsweise neutrales Biopolysaccharid, bevorzugt Dextran und/oder dessen Derivate oder Salze, insbesondere in einer Menge im Bereich von 0,1 bis 50 Gew.-%, insbesondere 1 bis 40 Gew.-%, vorzugsweise 5 bis 30 Gew.-%, bevorzugt 10 bis 20 Gew.-%, besonders bevorzugt 13 bis 18 Gew.-%, bezogen auf die Zusammensetzung;
   (e) gegebenenfalls mindestens ein chemisches Puffersystem ("Komponente (e)"), insbesondere in Form von Puffersalz(en), insbesondere in einer Menge im Bereich von 0,001 bis 5 Gew.-%, insbesondere 0,01 bis 4 Gew.-%, vorzugsweise 0,1 bis 3 Gew.-%, bevorzugt 0,5 bis 2 Gew.-%, besonders bevorzugt 1 bis 1,5 Gew.-%, bezogen auf die Zusammensetzung und berechnet als Summe aller Bestandteile des chemischen Puffersystems;
   (f) gegebenenfalls mindestens ein Blockier- und/oder Stabilisierungsmittel ("Komponente (f)"), insbesondere in einer Konzentration im Bereich von 0,001 µg/µl bis 100 µg/µl, insbesondere 0,005 µg/µl bis 80 µg/µl, vorzugsweise 0,01 µg/µl bis 40 µg/µl, bevorzugt 0,05 µg/µl bis 20 µg/µl, besonders bevorzugt 0,1 µg/µl bis 10 µg/µl, bezogen auf die Zusammensetzung, bezogen auf die Zusammensetzung;
   (g) gegebenenfalls mindestens ein anorganisches Salz ("Komponente (g)"), insbesondere Alkali- und/oder Erdalkali-Salz, vorzugsweise Alkali- und/oder Erdalkali-Chlorid, besonders bevorzugt Natriumchlorid, insbesondere in einer Menge im Bereich von 0,01 bis 15 Gew.-%, insbesondere 0,05 bis 10 Gew.-%, vorzugsweise 0,1 bis 10 Gew.-%, bevorzugt 0,5 bis 5 Gew.-%, besonders bevorzugt 1 bis 3 Gew.-%, bezogen auf die Zusammensetzung;
   (h) gegebenenfalls mindestens ein Detergens und/oder Tensid ("Komponente (h)"), insbesondere in einer Menge im Bereich von 0,001 bis 5 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,12 bis 1 Gew.-%, bezogen auf die Zusammensetzung.
10. Zusammensetzung, insbesondere Zusammensetzung zur Verwendung bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en), vorzugsweise Zusammensetzung nach einer der vorangehenden Ausführungsformen,
   wobei die Zusammensetzung enthält:
   (a) mindestens eine, vorzugsweise lokusspezifische, Hybridisierungssonde ("Komponente (a)"), insbesondere in einer Konzentration im Bereich von 0,1 ng/µl bis 50 ng/µl, insbesondere 0,5 ng/µl bis 50 ng/µl, vorzugsweise 0,7 ng/µl bis 8 ng/µl, bevorzugt 1 ng/µl bis 5 ng/µl, bezogen auf die Zusammensetzung;
   (b) mindestens ein polares protisches oder polares aprotisches Lösemittel ("Komponente (b)"), vorzugsweise mit cyclischer Molekülstruktur, bevorzugt γ-Butyrolacton, 2-Pyrrolidon (γ-Butyrolactam) und/oder Ethylencarbonat, insbesondere in einer Menge im Bereich von 0,5 bis 40 Vol.-%, insbesondere 1 bis 35 Vol.-%, vorzugsweise 2 bis 30 Vol.-%, bevorzugt 5 bis 20 Vol.-%, besonders bevorzugt 7 bis 15 Vol.-%, bezogen auf die Zusammensetzung;
   (c) Formamid und/oder dessen Salze ("Komponente (c)"), insbesondere in einer Menge im Bereich von 10 bis 60 Vol.-%, insbesondere 15 bis 50 Vol.-%, vorzugsweise 17 bis 40 Vol.-%, bevorzugt 20 bis 30 Vol.-%, bezogen auf die Zusammensetzung;
   (d) gegebenenfalls Dextran und/oder dessen Derivate oder Salze ("Komponente (d)"), insbesondere in einer Menge im Bereich von 0,1 bis 50 Gew.-%, insbesondere 1 bis 40 Gew.-%, vorzugsweise 5 bis 30 Gew.-%, bevorzugt 10 bis 20 Gew.-%, besonders bevorzugt 13 bis 18 Gew.-%, bezogen auf die Zusammensetzung;
   (e) gegebenenfalls mindestens ein chemisches Puffersystem, insbesondere in Form von Puffersalz(en), vorzugsweise Citrat und/oder Natriumchlorid, ("Komponente (e)"), insbesondere in einer Menge im Bereich von 0,001 bis 5 Gew.-%, insbesondere 0,01 bis 4 Gew.-%, vorzugsweise 0,1 bis 3 Gew.-%, bevorzugt 0,5 bis 2 Gew.-%, besonders bevorzugt 1 bis 1,5 Gew.-%, bezogen auf die Zusammensetzung und berechnet als Summe aller Bestandteile des chemischen Puffersystems;
   (f) gegebenenfalls mindestens ein Blockier- und/oder Stabilisierungsmittel ("Komponente (f)"), insbesondere in einer Konzentration im Bereich von 0,001 µg/µl bis 100 µg/µl, insbesondere 0,005 µg/µl bis 80 µg/µl, vorzugsweise 0,01 µg/µl bis 40 µg/µl, bevorzugt 0,05 µg/µl bis 20 µg/µl, besonders bevorzugt 0,1 µg/µl bis 10 µg/µl, bezogen auf die Zusammensetzung, bezogen auf die Zusammensetzung;
   (g) gegebenenfalls mindestens ein anorganisches Salz ("Komponente (g)"), insbesondere Alkali- und/oder Erdalkali-Salz, vorzugsweise Alkali- und/oder Erdalkali-Chlorid, besonders bevorzugt Natriumchlorid, insbesondere in einer Menge im Bereich von 0,01 bis 15 Gew.-%, insbesondere 0,05 bis 10 Gew.-%, vorzugsweise 0,1 bis 10 Gew.-%, bevorzugt 0,5 bis 5 Gew.-%, besonders bevorzugt 1 bis 3 Gew.-%, bezogen auf die Zusammensetzung; und
   (h) gegebenenfalls mindestens einen Polyalkylenglycolether ("Komponente (h)"), insbesondere in einer Menge im Bereich von 0,001 bis 5 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,12 bis 1 Gew.-%, bezogen auf die Zusammensetzung.
11. Verwendung einer Zusammensetzung nach einer der vorangehenden Ausführungsformen bei der Hybridisierung, insbesondere In-Situ-Hybridisierung, vorzugsweise automatisierten In-Situ-Hybridisierung, insbesondere zum Nachweis und/oder zur Detektion von Nukleinsäuren, bevorzugt RNA und/oder DNA, in einer biologischen Probe, vorzugsweise in einer oder mehreren Zelle(n) und/oder in einem oder mehreren Zellkern(en).
12. Verfahren zur Detektion von Nukleinsäuren, bevorzugt RNA und/oder DNA, und/oder von Chromosomenaberrationen in einer biologischen Probe, vorzugsweise in einer oder mehreren Zelle(n) und/oder in einem oder mehreren Zellkern(en) mittels Hybridisierung, insbesondere In-Situ-Hybridisierung, vorzugsweise automatisierter In-Situ-Hybridisierung, unter Verwendung einer Zusammensetzung nach einer der Ausführungsformen 1 bis 10.
13. Verfahren zur Detektion von Nukleinsäuren, bevorzugt RNA und/oder DNA, und/oder von Chromosomenaberrationen in einer biologischen Probe, vorzugsweise in einer oder mehreren Zelle(n) und/oder in einem oder mehreren Zellkern(en) mittels Hybridisierung, insbesondere In-Situ-Hybridisierung, vorzugsweise automatisierter In-Situ-Hybridisierung, insbesondere Verfahren nach Ausführungsform 12,
   wobei das Verfahren die folgenden Verfahrensschritte umfasst:
   (a) Bereitstellen einer biologischen Probe, insbesondere auf Basis von einer oder mehreren Zelle(n) und/oder einem oder mehreren Zellkern(en), vorzugsweise in Form von Gewebe, für die In-Situ-Hybridisierung;
   (b) Bereitstellen einer Zusammensetzung zur Verwendung bei der Hybridisierung, wobei die Zusammensetzung mindestens eine, vorzugsweise lokusspezifische, Hybridisierungssonde ("Komponente (a)"), mindestens ein polares protisches oder polares aprotisches Lösemittel, vorzugsweise mit cyclischer Molekülstruktur, ("Komponente (b)"), und mindestens ein Carbonsäureamid und/oder dessen Salze, insbesondere Formamid und/oder dessen Salze, in einer Menge von mehr als 10 Vol.-%, bezogen auf die Zusammensetzung ("Komponente (c)"), enthält, insbesondere Zusammensetzung nach einer der Ausführungsformen 1 bis 10;
   (c) Inkontaktbringen der biologischen Probe aus Verfahrensschritt (a) mit der Zusammensetzung aus Verfahrensschritt (b);
   (d) Denaturierung der biologischen Probe aus Verfahrensschritt (a) und der Zusammensetzung aus Verfahrensschritt (b), wobei die biologische Probe und die Zusammensetzung getrennt voneinander, insbesondere vor Durchführung von Verfahrensschritt (c), oder gemeinsam, insbesondere nach Durchführung von Verfahrensschritt (c), denaturiert werden;
   (e) nachfolgende Hybridisierung der in der Zusammensetzung enthaltenen mindestens einen, vorzugsweise lokusspezifischen, Hybridisierungssonde und den in den biologischen Proben enthaltenen Nukleinsäuren;
   (f) nachfolgende Detektion der hybridisierten, vorzugsweise lokusspezifischen, Hybridisierungssonden und/oder der zu detektierenden Nukleinsäuren in der biologischen Probe.
14. Verfahren nach Ausführungsform 13,
   wobei das Verfahren auf einem Automaten und/oder automatisch durchgeführt wird; und/oder
   wobei die Zusammensetzung in Verfahrensschritt (c) in einer Menge im Bereich von 0,01 bis 5.000 µl, insbesondere 0,1 bis 2.500 µl, vorzugsweise 1 bis 1.500 µl, bevorzugt 2 bis 1.000 µl, besonders bevorzugt 1 bis 500 µl, ganz besonders bevorzugt 20 bis 250 µl, noch mehr bevorzugt 40 bis 150 µl, eingesetzt wird; und/oder
   wobei Verfahrensschritt (e) unter Bewegung, insbesondere wellenförmiger und/oder kontinuierlicher Bewegung, durchgeführt wird; und/oder
   wobei Verfahrensschritt (e) mit kurzen oder mit langen Hybridisierungsdauern durchgeführt wird und/oder wobei Verfahrensschritt (e) über eine Dauer im Bereich von 10 min bis 240 min, insbesondere im Bereich von 30 min bis 180 min, vorzugsweise im Bereich von 60 min bis 150 min, bevorzugt im Bereich von 90 min bis 130 min, durchgeführt wird oder wobei Verfahrensschritt (e) über eine Dauer im Bereich von 1 h bis 100 h, insbesondere 2 h bis 80 h, vorzugsweise 3 h bis 50 h, bevorzugt 4 h bis 30 h, besonders bevorzugt 5 h bis 25 h, noch mehr bevorzugt 8 h bis 20 h, durchgeführt wird.
15. Kit (Kit-of-parts oder System) zur Detektion von Nukleinsäuren, bevorzugt RNA und/oder DNA, und/oder von Chromosomenaberrationen in einer biologischen Probe, vorzugsweise in einer oder mehreren Zelle(n) und/oder in einem oder mehreren Zellkern(en) mittels Hybridisierung, insbesondere In-Situ-Hybridisierung, vorzugsweise automatisierter In-Situ-Hybridisierung, wobei das Kit eine Zusammensetzung nach einer der Ausführungsformen 1 bis 10 enthält und/oder wobei das Kit zur Durchführung eines Verfahrens gemäß einer der Ausführungsformen 12 bis 14 bestimmt ist und/oder eingesetzt wird.

## Patentansprüche

1. Zusammensetzung zur Verwendung bei der Hybridisierung, wobei die Zusammensetzung enthält:
(a) mindestens eine Hybridisierungssonde ("Komponente (a)");
(b) mindestens ein polares protisches oder polares aprotisches Lösemittel ("Komponente (b)") mit cyclischer Molekülstruktur; und
(c) mindestens ein Carbonsäureamid und/oder dessen Salze ("Komponente (c)") in einer Menge von mehr als 10 Vol.-% bezogen auf die Zusammensetzung.

2. Zusammensetzung nach Anspruch 1, wobei die Zusammensetzung das mindestens eine polare protische oder polare aprotische Lösemittel in einer Menge von mindestens 1 Vol.-% oder Gew.-% und von höchstens 15 Vol.-% oder Gew.-%, bezogen auf die Zusammensetzung enthält.

3. Zusammensetzung nach Anspruch 1 oder 2, wobei das mindestens eine Lösemittel mit cyclischer Molekülstruktur ein polares protisches Lösemittel ist, insbesondere ausgewählt aus protischen cyclischen Amiden (Lactamen).

4. Zusammensetzung nach einem der vorangehenden Ansprüche, wobei die Zusammensetzung das mindestens eine Carbonsäureamid und/oder dessen Salze, insbesondere Formamid und/oder dessen Salze, in einer Menge von mehr als 10 Vol.-% und von höchstens 40 Vol.-%, bezogen auf die Zusammensetzung enthält.

5. Zusammensetzung nach einem der vorangehenden Ansprüche, wobei die Zusammensetzung die Komponente (b) und die Komponente (c) bzw. das mindestens eine polare protische oder polare aprotische Lösemittel und das mindestens eine Carbonsäureamid und/oder dessen Salze, insbesondere Formamid und/oder dessen Salze, in einem volumenbezogenen Verhältnis im Bereich von 1 : 10 bis 5 : 1 enthält.

6. Zusammensetzung nach einem der vorangehenden Ansprüche, wobei die Zusammensetzung mindestens ein Polysaccharid ("Komponente (d)"), bevorzugt Dextran, und/oder dessen Derivate oder Salze, besonders bevorzugt Dextransulfat, enthält, insbesondere wobei die Zusammensetzung die Komponente (d) in einer Menge im Bereich von 0,1 bis 50 Gew.-% bezogen auf die Zusammensetzung enthält.

7. Zusammensetzung nach einem der vorangehenden Ansprüche, wobei die Zusammensetzung mindestens ein Detergens und/oder Tensid enthält.

8. Zusammensetzung nach einem der vorangehenden Ansprüche, wobei die Zusammensetzung für In-Situ-Hybridisierungen im Zusammenhang mit der Diagnose und/oder Prognose von Erkrankungen eingesetzt wird, und die in diesem Zusammenhang zu untersuchenden Gene ausgewählt sind aus der Gruppe von ALK, ROS1, RET, NRG1, NTRK1, CARS, EML4, FGFR2, FGFR3, KIF5B, TGF, BCR, ABL, ALK, BCL2, BCL6, BIRC3, CCND1, EGR1, ETV6, FGFR1, FGFR3, IGH, KMT2A, MYC, PML, RARA, RUNX1, RUNX1T1, EWSR1, CHOP, FUS, COL1A1, DDIT3, JAZF1, NR4A3, FOXO1, FUS, PAX3, PAX7, PDGFB, SS18, TFE3, USP6, WT1, HER2/ERBB2, FGFR1, ALK, CCND1, CDK4, CD274, PDCD1LG2, EGR1, EGFR, ESR1, ETV1, FGF3,4,19, FGFR2, FGFR3, FHIT (RCC), KRAS, MDM2, MDM4, MET, MYB, MYC, MYCN, PIK3CA, PTEN, SMARCB1, SOX2, TERT, TOP2A, TP53, TYMS und/oder VHL.

9. Zusammensetzung zur Verwendung bei der Hybridisierung, vorzugsweise Zusammensetzung nach einem der vorangehenden Ansprüche, wobei die Zusammensetzung enthält:
(a) mindestens eine Hybridisierungssonde ("Komponente (a)");
(b) mindestens ein polares protisches oder polares aprotisches Lösemittel ("Komponente (b)") mit cyclischer Molekülstruktur;
(c) mindestens ein Carbonsäureamid und/oder dessen Salze ("Komponente (c)"), insbesondere Formamid und/oder dessen Salze, insbesondere in einer Menge im Bereich von 15 bis 50 Vol.-% bezogen auf die Zusammensetzung;
(d) gegebenenfalls mindestens ein Polysaccharid ("Komponente (d)");
(e) gegebenenfalls mindestens ein chemisches Puffersystem ("Komponente (e)");
(f) gegebenenfalls mindestens ein Blockier- und/oder Stabilisierungsmittel ("Komponente (f)");
(g) gegebenenfalls mindestens ein anorganisches Salz ("Komponente (g)");
(h) gegebenenfalls mindestens ein Detergens und/oder Tensid ("Komponente (h)").

10. Zusammensetzung zur Verwendung bei der Hybridisierung, vorzugsweise Zusammensetzung nach einem der vorangehenden Ansprüche, wobei die Zusammensetzung enthält:
(a) mindestens eine Hybridisierungssonde ("Komponente (a)");
(b) mindestens ein polares protisches oder polares aprotisches Lösemittel ("Komponente (b)") mit cyclischer Molekülstruktur;
(c) Formamid und/oder dessen Salze ("Komponente (c)");
(d) gegebenenfalls Dextran und/oder dessen Derivate oder Salze ("Komponente (d)");
(e) gegebenenfalls mindestens ein chemisches Puffersystem;
(f) gegebenenfalls mindestens ein Blockier- und/oder Stabilisierungsmittel ("Komponente (f)");
(g) gegebenenfalls mindestens ein anorganisches Salz ("Komponente (g)"); und
(h) gegebenenfalls mindestens einen Polyalkylenglycolether ("Komponente (h)").

11. Verwendung einer Zusammensetzung nach einem der vorangehenden Ansprüche bei der Hybridisierung.

12. Verfahren zur Detektion von Nukleinsäuren und/oder von Chromosomenaberrationen in einer biologischen Probe mittels Hybridisierung unter Verwendung einer Zusammensetzung nach einem der Ansprüche 1 bis 10.

13. Verfahren zur Detektion von Nukleinsäuren und/oder von Chromosomenaberrationen in einer biologischen Probe, insbesondere Verfahren nach Anspruch 12, wobei das Verfahren die folgenden Verfahrensschritte umfasst:
(a) Bereitstellen einer biologischen Probe auf Basis von einer oder mehreren Zelle(n) und/oder einem oder mehreren Zellkern(en) für die In-Situ-Hybridisierung;
(b) Bereitstellen einer Zusammensetzung zur Verwendung bei der Hybridisierung, wobei die Zusammensetzung mindestens eine Hybridisierungssonde ("Komponente (a)"), mindestens ein polares protisches oder polares aprotisches Lösemittel mit cyclischer Molekülstruktur, ("Komponente (b)"), und mindestens ein Carbonsäureamid und/oder dessen Salze, insbesondere Formamid und/oder dessen Salze, in einer Menge von mehr als 10 Vol.-% bezogen auf die Zusammensetzung ("Komponente (c)"), enthält, insbesondere Bereitstellen einer Zusammensetzung nach einem der Ansprüche 1 bis 10;
(c) Inkontaktbringen der biologischen Probe aus Verfahrensschritt (a) mit der Zusammensetzung aus Verfahrensschritt (b);
(d) Denaturierung der biologischen Probe aus Verfahrensschritt (a) und der Zusammensetzung aus Verfahrensschritt (b), wobei die biologische Probe und die Zusammensetzung getrennt voneinander, insbesondere vor Durchführung von Verfahrensschritt (c), oder gemeinsam, insbesondere nach Durchführung von Verfahrensschritt (c), denaturiert werden;
(e) nachfolgende Hybridisierung der in der Zusammensetzung enthaltenen mindestens einen Hybridisierungssonde und den in den biologischen Proben enthaltenen Nukleinsäuren;
(f) nachfolgende Detektion der hybridisierten Hybridisierungssonden und/oder der zu detektierenden Nukleinsäuren in der biologischen Probe.

14. Verfahren nach Anspruch 13, wobei das Verfahren auf einem Automaten und/oder automatisch durchgeführt wird und/oder wobei Verfahrensschritt (e) über eine Dauer im Bereich von 10 min bis 240 min durchgeführt wird.

15. Kit (Kit-of-parts oder System) zur Detektion von Nukleinsäuren und/oder von Chromosomenaberrationen in einer biologischen Probe, vorzugsweise in einer oder mehreren Zelle(n) und/oder in einem oder mehreren Zellkern(en) mittels Hybridisierung, wobei das Kit eine Zusammensetzung nach einem der Ansprüche 1 bis 10 enthält und/oder wobei das Kit zur Durchführung eines Verfahrens gemäß einem der Ansprüche 12 bis 14 bestimmt ist und/oder eingesetzt wird.
